# EUROPEAN PATENT SPECIFICATION

(11) **EP 2 883 858 B1**
(45) Date of publication and mention of the grant of the patent: **27.12.2017**
(21) Application number: 13827378.4
(22) Date of filing: 31.07.2013
(51) Int. Cl.: C07C 41/48, C07C 43/315, C07C 67/31, C07C 69/734, C07D 307/20, C07D 493/04, C07B 53/00, C07B 61/00

(54) **METHOD FOR PRODUCING HEXAHYDROFUROFURANOL DERIVATIVE**
VERFAHREN ZUR HERSTELLUNG EINES HEXAHYDROFUROFURANOLDERIVATS
PROCÉDÉ DE FABRICATION DE DÉRIVÉ D'HEXAHYDROFUROFURANOL

(30) Priority: 09.08.2012 JP 2012177296
(43) Date of publication of application: 17.06.2015
(73) Proprietor: Sumitomo Chemical Company Limited, Tokyo 104-8260 (JP)
(72) Inventor: AIKAWA, Toshiaki, Osaka-shi Osaka 555-0021 (JP); TAKEDA, Masahiro, Osaka-shi Osaka 555-0021 (JP); MIKI, Takashi, Osaka-shi Osaka 555-0021 (JP); IKEMOTO, Tetsuya, Osaka-shi Osaka 555-0021 (JP)
(74) Representative: J A Kemp
(86) International application number: PCT/JP2013/071291
(87) International publication number: WO 2014/024898

(56) References cited:
- WO-A1-2004/002975
- JP-A- 2004 107 315
- JP-A- 2005 502 707
- JP-A- 2007 131 613

## Description

### TECHNICAL FIELD

The present invention relates to a method for producing a hexahydrofurofuranol derivative useful as an intermediate in medicament synthesis, compounds useful as intermediates of the derivative, and methods for producing the intermediates.

### BACKGROUND ART

The compound represented by formula (VII) below, i.e., an optically active hexahydrofurofuranol derivative, is a useful intermediate in synthesis of a compound being an anti-AIDS medicament (WO 01/25240).

A method disclosed in WO 01/25240, EP-539192 A, WO 2004/002975, or Tetrahedron Letters, vol. 36, p. 505, 1995 is known for synthesizing a racemate of the compound of formula (VII). The method, however, requires ozone oxidation or use of tributyltin hydride and other compounds having high toxicity, which is industrially disadvantageous. To obtain an optically active form of the compound, optical resolution using enzymes is employed, in which one of the obtained enantiomers is discarded, leading to inefficiency.

A method for directly synthesizing the compound represented by formula (VII) is disclosed in Tetrahedron Letters, vol. 42, p. 4653, 2001. This method uses an organoselenium compound and thus cannot be considered as an industrial method.

A method using an optically active form of the compound as a material is disclosed in WO 2004/033462. The optically active form being a material is, however, expensive to cause an economical problem.

Furthermore, a method of providing the compound represented by formula (VII) is disclosed in JP 2007-131613 A, in which the diastereomer ratio is approximately 4:1. This method thus requires purification process including oxidizing a mixture of the compound represented by formula (VII) and the diastereomer thereof and further reducing the obtained product.

### SUMMARY OF THE INVENTION

The present invention solves the problems of conventional production methods (for example, use of ozone oxidation or reagents having strong toxicity, low diastereoselectivity). The present invention thus provides a method for producing efficiently and inexpensively on an industrial scale the compound represented by formula (VII) and having a good diastereomer ratio and enantiomer excess; intermediates useful in this method, and methods for producing the intermediates; and a method for producing a compound represented by formula (VIII).

The present invention, as described below, includes allowing a compound represented by general formula (I) or a polymer thereof to react with a compound represented by general formula (II) in the presence of an optically active amine catalyst, preferably in the presence of an optically active amine catalyst represented by general formula (IX) to give a compound represented by general formula (III); further acetalizing the compound represented by general formula (III) to give a compound represented by general formula (IV); further reducing the ester part of the compound represented by general formula (IV) to give compound (V); and preferably first cyclizing the compound (V) to give compound (VI) and then deprotecting the hydroxyl group of compound (VI) to provide the compound represented by general formula (VII) having a good diastereomer ratio and enantiomer excess. The diastereomer and enantiomer of compound (VII), which exist in small amounts, can be greatly reduced by protection of the hydroxyl group of compound (VII) to provide compound (VIII) and subsequent crystallization of compound (VIII).

Specifically, the present invention is as follows.
[1] A method for producing a compound represented by formula (V) (hereinafter also referred to as compound (V)), 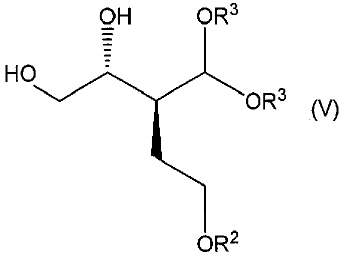 (wherein R² represents a protective group of a hydroxyl group, and R³ represents a C₁₋₁₂ alkyl group, C₂₋₁₂ alkenyl group, C₂₋₁₂ alkynyl group, or C₇₋₁₄ aralkyl group),
   the method including the steps of:
   allowing a compound represented by formula (I) (hereinafter also referred to as compound (I)), 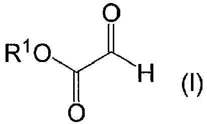 (wherein R¹ represents a protective group of a carboxyl group),
   or a polymer thereof to react with a compound represented by formula (II) (hereinafter also referred to as compound (II)), 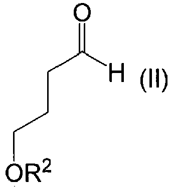 (wherein R² has the same meaning as defined above)
   in the presence of an optically active amine catalyst to give a compound represented by formula (III) (hereinafter also referred to as compound (III)), 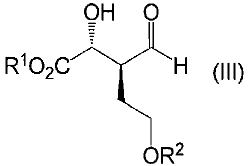 (wherein R¹ and R² have the same meaning as defined above);
   acetalizing the compound represented by formula (III) to give a compound represented by formula (IV) (hereinafter also referred to as compound (IV)), 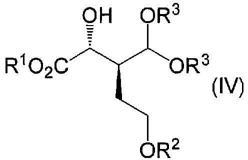 (wherein R¹, R², and R³ have the same meaning as defined above) ; and
   reducing the compound represented by formula (IV) to give the compound represented by formula (V).
[2] A method for producing a compound represented by formula (VII) (hereinafter also referred to as compound (VII)), 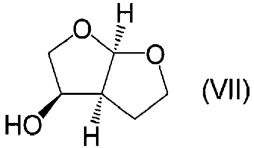 the method including the steps of:
   allowing a compound represented by formula (I), 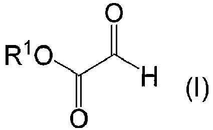 (wherein R¹ represents a protective group of a carboxyl group),
   or a polymer thereof to react with a compound represented by formula (II), 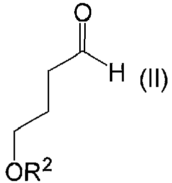 (wherein R² represents a protective group of a hydroxyl group)
   in the presence of an optically active amine catalyst to give a compound represented by formula (III), 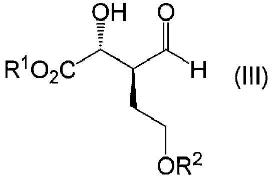 (wherein R¹ and R² have the same meaning as defined above);
   acetalizing the compound represented by formula (III) to give a compound represented by formula (IV), 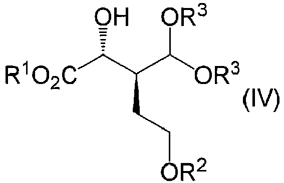 (wherein R³ represents a C₁₋₁₂ alkyl group, C₂₋₁₂ alkenyl group, C₂₋₁₂ alkynyl group, or C₇₋₁₄ aralkyl group, and R¹ and R² have the same meaning as defined above);
   reducing the compound represented by formula (IV) to give a compound represented by formula (V), 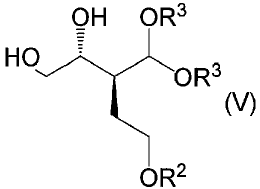 (wherein R² and R³ have the same meaning as defined above) ; and
   deprotecting the acetalized formyl group represented by CH(OR³)₂ and the R²-protected hydroxyl group in the compound represented by formula (V) so that the obtained product is cyclized to give the compound represented by formula (VII).
[3] A production method according to [2] comprising the steps of:
   producing a compound represented by formula (V) according to [2];
   deprotecting the acetalized formyl group represented by CH(OR³)₂ in the compound represented by formula (V) so that the obtained product is cyclized to give a compound represented by formula (VI), 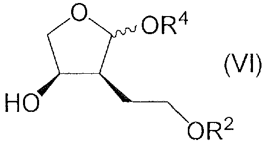 wherein R⁴ represents a C₁₋₁₂ alkyl group, C₂₋₁₂ alkenyl group, C₂₋₁₂ alkynyl group, or C₇₋₁₄ aralkyl group, and R² has the same meaning as defined above; and
   deprotecting the R²-protected hydroxyl group in the compound represented by formula (VI) so that the obtained product is cyclized to give the compound represented by formula (VII).
[4] A method for producing a compound represented by formula (VI) (hereinafter also referred to as compound (VI)), 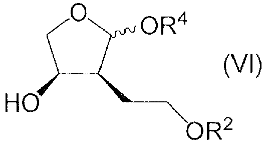 (wherein R⁴ represents a C₁₋₁₂ alkyl group, C₂₋₁₂ alkenyl group, C₂₋₁₂ alkynyl group, or C₇₋₁₄ aralkyl group, and R² represents a protective group of a hydroxyl group),
   the method including the steps of:
   allowing a compound represented by formula (I), 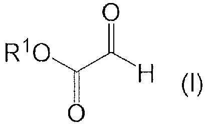 (wherein R¹ represents a protective group of a carboxyl group), or a polymer thereof to react with a compound represented by formula (II), 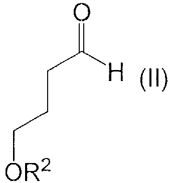 (wherein R² has the same meaning as defined above) in the presence of an optically active amine catalyst to give a compound represented by formula (III), 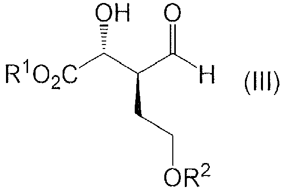 (wherein R¹ and R² have the same meaning as defined above);
   acetalizing the compound represented by formula (III) to give a compound represented by formula (IV), 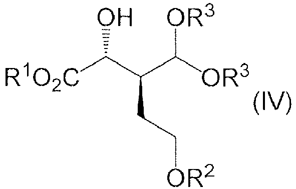 (wherein R³ represents a C₁₋₁₂ alkyl group, C₂₋₁₂ alkenyl group, C₂₋₁₂ alkynyl group, or C₇₋₁₄ aralkyl group, and R¹ and R² have the same meaning as defined above);
   reducing the compound represented by formula (IV) to give a compound represented by formula (V), 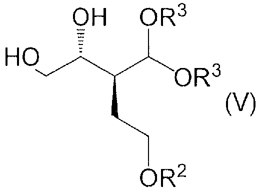 (wherein R² and R³ have the same meaning as defined above) ; and
   deprotecting the acetalized formyl group represented by CH(OR³)₂ in the compound represented by formula (V) so that the obtained product is cyclized to give the compound represented by formula (VI).
[5] A method for producing a compound represented by formula (VIII) (hereinafter also referred to as compound (VIII)), 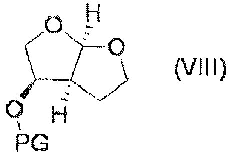 (wherein PG represents a protective group of a hydroxyl group),
   the method including the steps of:
   allowing a compound represented by formula (I), 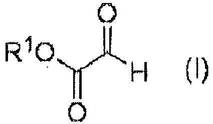 (wherein R¹ represents a protective group of a carboxyl group),
   or a polymer thereof to react with a compound represented by formula (II), 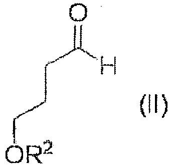 (wherein R² represents a protective group of a hydroxyl group) in the presence of an optically active amine catalyst to give a compound represented by formula (III), 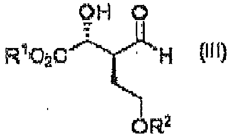 (wherein R¹ and R² have the same meaning as defined above);
   acetalizing the compound represented by formula (III) to give a compound represented by formula (IV), 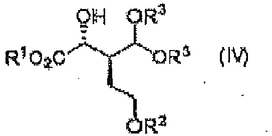 (wherein R³ represents a C₁₋₁₂ alkyl group, C₂₋₁₂ alkenyl group, C₂₋₁₂ alkynyl group, or C₇₋₁₄ aralkyl group, and R¹ and R² have the same meaning as defined above);
   reducing the compound represented by formula (IV) to give a compound represented by formula (V), 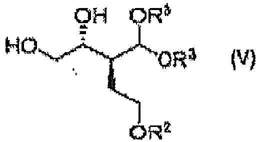 (wherein R² and R³ have the same meaning as defined above);
   deprotecting the acetalized formyl group represented by CH(OR³)₂ in the compound represented by formula (V) so that the obtained product is cyclized to give a compound represented by formula (VI), 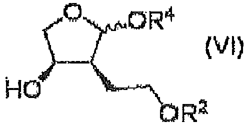 (wherein R⁴ represents a C₁₋₁₂ alkyl group, C₂₋₁₂ alkenyl group, C₂₋₁₂ alkynyl group, or C₇₋₁₄ aralkyl group, and R² has the same meaning as defined above);
   deprotecting the R²-protected hydroxyl group in the compound represented by formula (VI) so that the obtained product is cyclized to give a compound represented by formula (VII); and 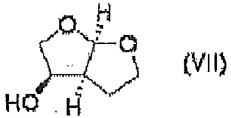
   protecting the hydroxyl group of the compound represented by formula (VII) to give the compound represented by formula (VIII).
[6] The production method according to any one of [1] to [5] above, wherein the optically active amine catalyst is a compound represented by formula (IX) (hereinafter also referred to as compound (IX)), 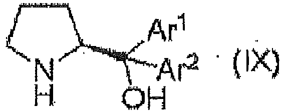 (wherein Ar¹ and Ar² each independently represent a C₁₋₁₂ alkyl group, C₂₋₁₂ alkenyl group, C₂₋₁₂ alkynyl group, C₇₋₁₄ aralkyl group, or optionally substituted phenyl group).
[7] A compound represented by formula (III), 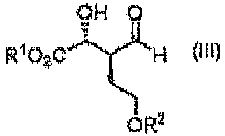 (wherein R¹ represents a protective group of a carboxyl group, and R² represents a protective group of a hydroxyl group).
[8] The compound according to [7] above, wherein R¹ is an ethyl group and R² is a benzyl group.
[9] A compound represented by formula (IV), 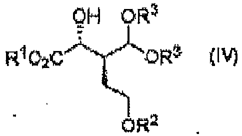 (wherein R¹ represents a protective group of a carboxyl group, R² represents a protective group of a hydroxyl group, and R³ represents a C₁₋₁₂ alkyl group, C₂₋₁₂ alkenyl group, C₂₋₁₂ alkynyl group, or C₇₋₁₄ aralkyl group) .
[10] The compound according to [9] above, wherein R¹ is an ethyl group, R² is a benzyl group, and R³ is a methyl group.
[11] A compound represented by formula (V), 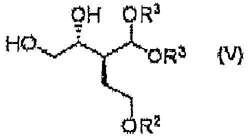 (wherein R² represents a protective group of a hydroxyl group, and R³ represents a C₁₋₁₂ alkyl group, C₂₋₁₂ alkenyl group, C₂₋₁₂ alkynyl group, or C₇₋₁₄ aralkyl group).
[12] The compound according to [11] above, wherein R² is a benzyl group and R³ is a methyl group.
[13] A compound represented by formula (VI), 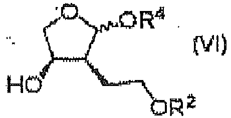 (wherein R² represents a protective group of a hydroxyl group, and R⁴ represents a C₁₋₁₂ alkyl group, C₂₋₁₂ alkenyl group, C₂₋₁₂ alkynyl group, or C₇₋₁₄ aralkyl group).
[14] The compound according to [13] above, wherein R² is a benzyl group and R⁴ is a methyl group.

### MODE FOR CARRYING OUT THE INVENTION

The present invention will be described below in detail.

As used herein, a "C₁₋₁₂ aliphatic hydrocarbon group" means a C₁₋₁₂ chain hydrocarbon group or C₃₋₁₂ alicyclic hydrocarbon group.

As used herein, the "C₁₋₁₂ chain hydrocarbon group" means a C₁₋₁₂ alkyl group, C₂₋₁₂ alkenyl group, or C₂₋₁₂ alkynyl group.

As used herein, the "C₁₋₁₂ alkyl group" means a straight or branched chain alkyl group having 1 to 12 carbon atoms and examples therof include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, heptyl, octyl, nonyl, decyl, undecyl, and dodecyl. Among them, C₁₋₈ alkyl group is preferred, and C₁₋₄ alkyl group is particularly preferred.

As used herein, a "C₁₋₆ alkyl (group)" means a straight or branched chain alkyl (group) having 1 to 6 carbon atoms and examples thereof include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, and 2-ethylbutyl. Among them, C₁₋₄ alkyl (group) is preferred.

As used herein, the "C₁₋₄ alkyl group" means a straight or branched chain alkyl group having 1 to 4 carbon atoms and examples thereof include methyl, ethyl, propyl, isopropyl, butyl, isobutyl, sec-butyl, and tert-butyl.

As used herein, the "C₂₋₁₂ alkenyl group" means a straight or branched chain alkenyl group having 2 to 12 carbon atoms and examples thereof include ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, 5-hexenyl, 1-heptenyl, 1-octenyl, 1-nonenyl, 1-decenyl, 1-undecenyl, and 1-dodecenyl. Among them, C₂₋₈ alkenyl group is preferred, and C₂₋₄ alkenyl groups are particularly preferred.

As used herein, a "C₂₋₆ alkenyl (group)" means a straight or branched chain alkenyl (group) having 2 to 6 carbon atoms and examples thereof include ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, and 5-hexenyl. Among them, C₂₋₄ alkenyl (group) is particularly preferred.

As used herein, the "C₂₋₁₂ alkynyl group" means a straight or branched chain alkynyl group having 2 to 12 carbon atoms and examples thereof include ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, 1-heptynyl, 1-octynyl, 1-nonynyl, 1-decynyl, 1-undecynyl, and 1-dodecynyl. Among them, C₂₋₈ alkynyl group is preferred, and C₂₋₄ alkynyl group is particularly preferred.

As used herein, the "C₃₋₁₂ alicyclic hydrocarbon group" means a C₃₋₁₂ cycloalkyl group or C₄₋₁₂ cycloalkenyl group.

As used herein, the "C₃₋₁₂ cycloalkyl group" means a cyclic alkyl group having 3 to 12 carbon atoms and examples thereof include cyclopropyl, cyclobutyl, cyclopentyl, cyclohexyl, cycloheptyl, cyclooctyl, cyclononyl, cyclodecyl, cycloundecyl, and cyclododecyl. Among them, C₃₋₈ cycloalkyl group is preferred.

As used herein, the "C₄₋₁₂ cycloalkenyl group" means a cyclic alkenyl group having 4 to 12 carbon atoms and examples thereof include 2-cyclopenten-1-yl, 3-cyclopenten-1-yl, 2-cyclohexen-1-yl, 3-cyclohexen-1-yl, 2-cyclohepten-1-yl, 2-cycloocten-1-yl, 2-cyclononen-1-yl, 2-cyclodecen-1-yl, 2-cyclododecen-1-yl, 2,4-cyclopentadien-1-yl, 2, 4-cyclohexadien-1-yl, and 2,5-cyclohexadien-1-yl. Among them, C₄₋₈ cycloalkenyl group is preferred.

As used herein, a "C₆₋₁₂ aromatic hydrocarbon group (C₆₋₁₂ aryl group)" means an aromatic monocyclic or polycyclic (condensed) hydrocarbon group having 6 to 12 carbon atoms, and examples thereof include phenyl, 1-naphthyl, 2-naphthyl, acenaphthylene, and biphenylyl. Among them, C₆₋₁₀ aromatic hydrocarbon group (C₆₋₁₀ aryl group) is preferred.

As used herein, the "C₆₋₁₀ aryl (group)" means an aromatic monocyclic or polycyclic (condensed) hydrocarbon group having 6 to 10 carbon atoms, and examples thereof include phenyl, 1-naphthyl, and 2-naphthyl.

As used herein, the "C₇₋₁₄ aralkyl group" means a "C₁₋₄ alkyl group" substituted by a "C₆₋₁₀ aryl group" and examples thereof include benzyl, 1-phenylethyl, 2-phenylethyl, (naphthyl-1-yl)methyl, (naphthyl-2-yl)methyl, 1-(naphthyl-1-yl)ethyl, 1-(naphthyl-2-yl)ethyl, 2-(naphthyl-1-yl)ethyl, and 2-(naphthyl-2-yl)ethyl.

As used herein, examples of the substituents of the "optionally substituted phenyl group" include a C₁₋₁₂ alkyl group, a C₁₋₁₂ alkoxy group, a C₂₋₁₃ alkoxycarbonyl group, a C₁₋₁₂ fluorinated alkyl group, a C₁₋₁₂ fluorinated alkyloxy group, a C₂₋₁₃ acyl group, a nitro group, a cyano group, a protected amino group, and a halogen atom.

As used herein, the "halogen atom" means a fluorine atom, chlorine atom, bromine atom, or iodine atom.

As used herein, the "C₁₋₁₂ alkoxy group" means a straight or branched chain alkoxy group having 1 to 12 carbon atoms and examples thereof include methoxy, ethoxy, propoxy, isopropoxy, butoxy, isobutoxy, sec-butoxy, tert-butoxy, pentyloxy, isopentyloxy, neopentyloxy, hexyloxy, heptyloxy, octyloxy, nonyloxy, decyloxy, undecyloxy, and dodecyloxy. Among them, C₁₋₈ alkoxy group, particularly C₁₋₄ alkoxy group is preferred.

As used herein, the "C₁₋₆ alkoxy (group)" means a straight or branched chain alkoxy (group) having 1 to 6 carbon atoms and examples thereof include methoxy, ethoxy, propoxy, isopropoxy, butoxy, isobutoxy, sec-butoxy, tert-butoxy, pentyloxy, isopentyloxy, neopentyloxy, and hexyloxy. Among them, C₁₋₄ alkoxy (group) is preferred.

As used herein, the "C₂₋₁₃ alkoxycarbonyl group" means a group where a "C₁₋₁₂ alkoxy group" is bound to -C=O- and examples thereof include methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, isopentyloxycarbonyl, neopentyloxycarbonyl, hexyloxycarbonyl, heptyloxycarbonyl, octyloxycarbonyl, nonyloxycarbonyl, decyloxycarbonyl, undecyloxycarbonyl, and dodecyloxycarbonyl. Among them, C₂₋₉ alkoxycarbonyl group, particularly C₂₋₅ alkoxycarbonyl group is preferred.

As used herein, the "C₁₋₁₂ fluorinated alkyloxy group" means a "C₁₋₁₂ alkoxy group" where a hydrogen atom(s) is/are substituted by a fluorine atom(s) . The number of fluorine atoms is-not particularly limited, including perfluorosubstitution. Specific examples of the C₁₋₁₂ fluorinated alkyloxy group include fluoromethoxy, difluoromethoxy, trifluoromethoxy, 2-fluoroethoxy, 2,2-difluoroethoxy, 2,2,2-trifluoroethoxy, 3-fluoropropoxy, 4-fluorobutoxy, 5-fluoropentyloxy, 6-fluorohexyloxy, 7-fluoroheptyloxy, 8-fluorooctyloxy, 9-fluorononyloxy, 10-fluorodecyloxy, 11-fluoroundecyloxy, and 12-fluorododecyloxy.

As used herein, the "C₁₋₁₂ fluorinated alkyl group" means a "C₁₋₁₂ alkyl group" where a hydrogen atom(s) is/are substituted by a fluorine atom(s). The number of fluorine atoms is not particularly limited, including perfluorosubstitution. Typical examples of the C₁₋₁₂ fluorinated alkyl group include fluoromethyl, difluoromethyl, trifluoromethyl, 2-fluoroethyl, 2,2-difluoroethyl, 2,2,2-trifluoroethyl, 3-fluoropropyl, 4-fluorobutyl, 5-fluoropentyl, 6-fluorohexyl, 7-fluoroheptyl, 8-fluorooctyl, 9-fluorononyl, 10-fluorodecyl, 11-fluoroundecyl, and 12-fluorododecyl.

As used herein, the "C₂₋₁₃ acyl group" is an atomic group obtained by removing a hydroxyl group from a C₂₋₁₃ carboxylic acid, and means a "C₂₋₁₃ aliphatic acyl group" or "C₇₋₁₃ aromatic acyl group."

As used herein, the "C₂₋₁₃ aliphatic acyl group" means a group where a "C₁₋₁₂ aliphatic hydrocarbon group" is bound to -C(=O)-, and examples thereof include acetyl, propanoyl, butanoyl, 2-methylpropanoyl, pentanoyl, hexanoyl, heptanoyl, octanoyl, nonanoyl, decanoyl, undecanoyl, dodecanoyl, acryloyl, methacryloyl, crotonoyl, isocrotonoyl, propionoyl, cyclopentylcarbonyl, and cyclohexylcarbonyl. Among them, C₂₋₁₃ alkylcarbonyl group is preferred, and C₂₋₉ alkylcarbonyl group is particularly preferred.

As used herein, the "C₇₋₁₃ aromatic acyl group" means a group where a "C₆₋₁₂ aromatic hydrocarbon group (C₆₋₁₂ aryl group)" is bound to -C(=O)-, and examples thereof include benzoyl, 1-naphthoyl, and 2-naphthoyl.

As used herein, the "protected amino group" means an amino group protected by a "protective group." Examples of the "protective group" include C₁₋₆ alkyl groups, C₂₋₆ alkenyl groups, C₆₋₁₀ aryl groups, C₇₋₁₄ aralkyl groups, C₁₋₆ alkyl-carbonyl groups, C₁₋₆ alkoxy-carbonyl groups, C₂₋₆ alkenyl-oxycarbonyl groups, C₆₋₁₀ aryl-carbonyl groups, C₇₋₁₄ aralkyl-carbonyl groups, C₆₋₁₀ aryl-oxycarbonyl groups, C₇₋₁₄ aralkyl-oxycarbonyl groups, C₆₋₁₀ aryl sulfonyl groups, a benzhydryl group, a trityl group, tri-C₁₋₆ alkylsilyl groups, a 9-fluorenylmethyloxycarbonyl group, and a phthaloyl group. The above substituents are each optionally substituted by a halogen atom, C₁₋₆ alkyl group, C₁₋₆ alkoxy group, or nitro group.

Typical examples of the protective group include acetyl, trifluoroacetyl, pivaloyl, tert-butoxycarbonyl, 2,2,2-trichloroethoxycarbonyl, benzyloxycarbonyl, 9-fluorenylmethyloxycarbonyl, benzhydryl, trityl, phthaloyl, allyloxycarbonyl, p-toluenesulfonyl, and o-nitrobenzenesulfonyl.

As used herein, the "protective group of a carboxyl group" can be a C₁₋₆ alkyl group, a C₇₋₁₄ aralkyl group, a phenyl group; a trityl group, and a silyl group optionally substituted by a C₁₋₆ alkyl group.

Typical examples of the protective group include methyl, ethyl, tert-butyl, benzyl, trityl, trimethylsilyl, triethylsilyl, dimethylphenylsilyl, tert-butyldimethylsilyl, and tert-butyldiethylsilyl.

As used herein, the "protective group of a hydroxyl group" can be a C₁₋₆ alkyl groups, a phenyl group, a trityl group, a C₇₋₁₄ aralkyl group, a formyl group, a C₁₋₆ alkyl-carbonyl group, a benzoyl group, a C₇₋₁₄ aralkyl-carbonyl group (e.g., benzylcarbonyl), a 2-tetrahydropyranyl group, a 2-tetrahydrofuranyl group, a silyl group optionally substituted by a C₁₋₆ alkyl group, a succinimidoxycarbonyl group, a C₆₋₁₀ aryl-carbonyl group, a C₆₋₁₀ aryl-oxycarbonyl group, and a C₇₋₁₄ aralkyl-oxycarbonyl group. The above substituents are each optionally substituted by a halogen atom, C₁₋₆ alkyl group, C₁₋₆ alkoxy group, carboxyl group, nitro group, or other groups.

Typical examples of the protective group include benzyl, acetyl, trimethylsilyl, triethylsilyl, dimethylphenylsilyl, tert-butyldimethylsilyl, tert-butyldiethylsilyl, succinimidoxycarbonyl, phenoxycarbonyl, and benzyloxycarbonyl.

R¹ represents a protective group of a carboxyl group.

R¹ is preferably a C₁₋₈ alkyl group, more preferably a C₁₋₄ alkyl group, particularly preferably an ethyl group.

R² represents a protective group of a hydroxyl group.

R² is preferably a C₇₋₁₄ aralkyl group, particularly preferably a benzyl group.

R³ represents a C₁₋₁₂ alkyl group, C₂₋₁₂ alkenyl group, C₂₋₁₂ alkynyl group, or C₇₋₁₄ aralkyl group.

R³ is preferably a C₁₋₁₂ alkyl group, more preferably a C₁₋₈ alkyl group, still more preferably a C₁₋₄ alkyl group, particularly preferably a methyl group.

R⁴ represents a C₁₋₁₂ alkyl group, C₂₋₁₂ alkenyl group, C₂₋₁₂ alkynyl group, or C₇₋₁₄ aralkyl group.

R⁴ is preferably a C₁₋₁₂ alkyl group, more preferably a C₁₋₈ alkyl group, still more preferably a C₁₋₄ alkyl group, particularly preferably a methyl group.

PG represents a protective group of a hydroxy group.

PG is preferably a succinimidoxycarbonyl group, a C₆₋₁₀ aryl-oxycarbonyl group, or a C₆₋₁₀ aryl-carbonyl group (wherein the C₆₋₁₀ aryl-oxycarbonyl group or C₆₋₁₀ aryl-carbonyl group is optionally substituted by a methyl group, methoxy group, nitro group, carboxyl group, halogen atom, or others), particularly preferably a succinimidoxycarbonyl group.

Ar¹ and Ar² each independently represent a C₁₋₁₂ alkyl group, C₂₋₁₂ alkenyl group, C₂₋₁₂ alkynyl group, C₇₋₁₄ aralkyl group, or optionally substituted phenyl group.

Ar¹ and Ar² are each independently preferably a phenyl group, a phenyl group having a C₁₋₄ fluorinated alkyl group, or a phenyl group having a C₁₋₄ alkyl group, more preferably a phenyl group, trifluoromethylphenyl group, or tolyl group (methylphenyl group), still more preferably Ar¹ and Ar² are both phenyl groups, both 3,5-bis (trifluoromethyl) phenyl groups, or both 4-methylphenyl groups.

According to the present invention, the hexahydrofurofuranol derivative is produced by steps 1 to 6 below. 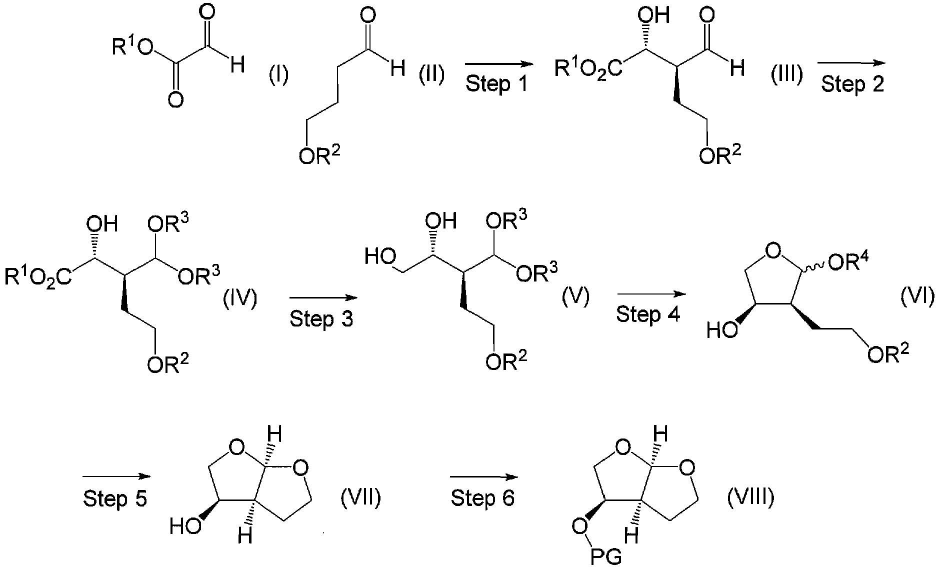 (wherein each symbol has the same meaning as defined above.)

Next, each step will be described.

Step 1: Compound (I) or its polymer and compound (II) are allowed to react in the presence of an optically active amine catalyst to give compound (III) (aldol reaction).

Compound (I) or its polymer can be a compound represented by general formula (I-1) below (hereinafter also referred to as polymer (I-1)) and a compound represented by general formula (I-2) (hereinafter also referred to as polymer (I-2)). 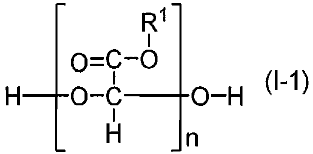 (wherein n represents an integer of 2 or more, and other symbols have the same meaning as defined above.) 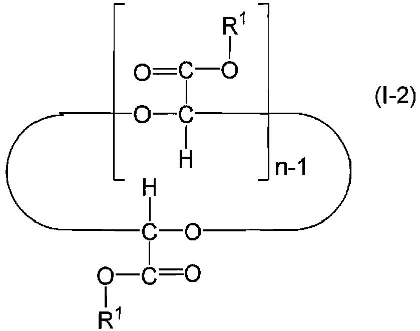 (wherein each symbol has the same meaning as defined above.)

Compound (I) is usually commercially available in the form of a solution of polymer (I-1) or polymer (I-2) in toluene. In this step, a commercial product can be used as it is because compound (I) is reactive even in using it in the form of polymers and the reaction can proceed even in toluene.

The amount of compound (II) used is preferably from 0.5 to 10 mol, more preferably from 0.5 to 5 mol per mole of compound (I) in terms of the yield, selectivity, and cost efficiency.

The optically active amine catalyst is preferably compound (IX), 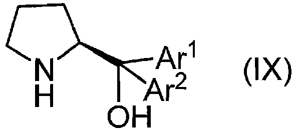 (wherein each symbol has the same meaning as defined above) in terms of the diastereoselectivity, depending on the type of compound (II). In particular, preferred are compounds where Ar¹ and Ar² each independently represent a phenyl group, a phenyl group having a C₁₋₄ fluorinated alkyl group, or a phenyl group having a C₁₋₄ alkyl group; more preferred are compounds where Ar¹ and Ar² each independently represent a phenyl group, trifluoromethylphenyl group, or tolyl group; and still more preferred are compounds where Ar¹ and Ar² are both phenyl groups, both 3,5-bis(trifluoromethyl)phenyl groups, or both 4-methylphenyl groups.

The amount of the optically active amine catalyst used is preferably from 0.1 to 30 mol%, more preferably from 0.5 to 15 mol%, particularly preferably from 1 to 10 mol % with respect to compound (I) in terms of the yield and cost efficiency.

The aldol reaction is preferably carried out in the presence of a solvent. Examples of the solvent used in the present invention include aromatic hydrocarbon solvents (e.g., toluene, benzene, xylene); aliphatic hydrocarbon solvents (e.g., hexane, heptane, cyclohexane); alcohol solvents (e.g., methanol, ethanol, 2-propanol, 2-methyl-2-propanol); halogenated hydrocarbon solvents (e.g., chloroform, dichloromethane, carbon tetrachloride, 1-chlorobutane); ether solvents (e.g., diethyl ether, tetrahydrofuran (THF), 2-methyltetrahydrofuran, 1,2-dimethoxyethane, tert-butyl methyl ether, tert-butyl ethyl ether, diisopropyl ether, dibutyl ether, cyclopentyl methyl ether, 1,4-dioxane, bis(2-methoxy ethyl)ether); ester solvents (e.g., ethyl acetate, isopropyl acetate); ketone solvents (e.g., acetone, 2-butanone, 3-pentanone); nitrile solvents (e.g., acetonitrile, propionitrile); aprotic polar solvents (e.g., dimethylformamide, dimethylacetamide, dimethyl sulfoxide); water; and mixed solvents thereof.

Among these solvents, aromatic hydrocarbon solvents; alcohol solvents; halogenated hydrocarbon solvents; ether solvents; nitrile solvents; water-; and mixed solvents thereof are preferred in terms of the enantioselectivity and diastereoselectivity, while the solvent depends on the type of compound (II). Furthermore, toluene, tetrahydrofuran, acetonitrile, water, or a mixed solvent containing at least one of these is preferably used; a solvent containing two of these is more preferably used; and a mixed solvent of toluene, tetrahydrofuran (THF), and water is particularly preferred in terms of excellent yield, enantioselectivity, and diastereoselectivity.

When this step is carried out in a solvent free of toluene, a commercially available solution of compound (I-1) or (I-2) in toluene is used after being concentrated.

The aldol reaction is carried out by a process of adding compound (II), an optically active amine catalyst, and a solvent to commercially available polymer (I-1) or polymer (I-2) and mixing these; a process of mixing commercially available polymer (I-1) or polymer (I-2), an optically active amine catalyst, and a solvent and adding compound (II) to the mixture, followed by mixing; or other processes. In terms of the yield and selectivity, the aldol reaction is preferably carried out by the process of mixing commercially available polymer (I-1) or polymer (I-2), an optically active amine catalyst, and a solvent and adding compound (II) to the mixture, followed by mixing.

The aldol reaction is carried out preferably in the range of from 0°C to 100°C, more preferably in the range of from 0°C to 40°C, depending on the type of compound (II)..

The reaction time is preferably from 1 to 100 hours, more preferably from 3 to 50 hours, particularly preferably from 5 to 20 hours, depending on the type of compound (II) and the reaction temperature.

The progress of the reaction can be determined by analytic means, such as thin layer chromatography, gas chromatography, and high performance liquid chromatography.

Compound (III) contained in the reaction mixture thus obtained can be isolated by subjecting the reaction mixture to conventional post-treatments (e.g., neutralization, extraction, water washing, distillation, and crystallization). Compound (III) can be purified by subjecting compound (III) to extraction purification treatment, distillation treatment, adsorption treatment with activated carbon, silica, alumina, and other materials, and chromatography treatment, such as silica gel column chromatography. Alternatively, compound (III) may be subjected to the next step as being in the reaction mixture.

In this step, the anti-form of compound (III) is preferentially obtained. The diastereoselectivity with a diastereomer ratio (syn/anti ratio) of 50/50 or more, preferably 20/80 or more, more preferably 10/90 or more is achieved.

Owing to the use of the optically active amine catalyst, the enantioselectivity with an enantiomer excess of 50 ee% or more, preferably 80 ee% or more, more preferably 90 ee% or more is achieved in this step.

In steps 2 to 6, this diastereomer ratio and enantiomer excess are substantially kept.

Step 2: Compound (III) is acetalized to give compound (IV) .

The acetalization reaction is carried out by allowing compound (III) to react with R³OH or HC(OR³)₃ (wherein R³ has the same meaning as defined above) in the presence of an acid catalyst.

Preferably, the acetalization is carried out in step 2A of allowing isolated unpurified compound (III) to react with R³OH in the presence of an acid catalyst, or step 2B of allowing a reaction mixture containing compound (III) after completion of the aldol reaction (step 1) to react with HC(OR³)₃ in the presence of an acid catalyst.

Step 2A will be described below.

The amount of R³OH used is preferably from 2 to 200 mol, more preferably from 10 to 100 mol per mole of compound (II) in terms of the yield and cost efficiency. In step 2A, R³OH also serves as a reaction solvent.

Examples of the acid catalyst used include inorganic proton acids, such as hydrochloric acid, sulfuric acid, and phosphoric acid; organic proton acids, such as methanesulfonic acid, pyridinium p-toluenesulfonate, p-toluenesulfonic acid or its hydrate (monohydrate), and acidic ion-exchange resin; and aprotic Lewis acids, such as boron trifluoride-THF complex, magnesium chloride, zinc chloride, and aluminum chloride. In terms of the reaction selectivity, sulfuric acid, methanesulfonic acid, pyridinium p-toluenesulfonate, p-toluenesulfonic acid or its hydrate (monohydrate) is preferred.

The amount of the acid catalyst used is preferably from 0.01 to 1 mol, more preferably from 0.01 to 0.1 mol per mole of compound (II) in terms of the reactivity and cost efficiency.

The acetalization reaction is carried out by a process of adding R³OH and an acid catalyst to isolated unpurified compound (III) and mixing these; or a process of adding an acid catalyst to isolated unpurified compound (III), and then adding R³OH to the mixture, followed by mixing; or other processes. In terms of easy operation, the acetalization reaction is preferably carried out by the process of adding R³OH and an acid catalyst to isolated unpurified compound (III) and mixing these.

The acetalization reaction is carried out preferably in the range of from 0°C to 100°C, more preferably in the range of from 20°C to 80°C, particularly preferably in the range of from 40°C to 60°C, depending on the types of R³OH and the acid catalyst.

The reaction time is preferably from 10 minutes to 50 hours, more preferably from 30 minutes to 20 hours, particularly preferably from 1 to 10 hours, depending on the types of R³OH and the acid catalyst, and the reaction temperature.

The progress of the reaction can be determined by analytic means, such as thin layer chromatography, gas chromatography, and high performance liquid chromatography.

Next, step 2B will be described.

The amount of HC(OR³)₃ used is preferably from 1 to 20 mol, more preferably 3 to 10 mol per mole of compound (II) in terms of the yield and cost efficiency.

Examples of the acid catalyst used include inorganic proton acids, such as hydrochloric acid, sulfuric acid, and phosphoric acid; organic proton acids, such as methanesulfonic acid, pyridinium p-toluenesulfonate, p-toluenesulfonic acid or its hydrate (monohydrate), and acidic ion-exchange resin; and aprotic Lewis acids, such as boron trifluoride-THF complex, magnesium chloride, zinc chloride, and aluminum chloride. In terms of the reaction selectivity, sulfuric acid, methanesulfonic acid, pyridinium p-toluenesulfonate, p-toluenesulfonic acid or its hydrate (monohydrate) is preferred.

The amount of the acid catalyst used is preferably from 0.01 to 1 mol, more preferably from 0.01 to 0.1 mol per mole of compound (II) in terms of the reaction rate.

The acetalization reaction is carried out by a process of adding a reaction mixture containing compound (III) after completion of the aldol reaction (step 1) to a mixture of HC(OR³)₃ and an acid catalyst, followed by mixing; or a process of adding an acid catalyst to a reaction mixture containing compound (III) after completion of the aldol reaction, and then adding HC(OR³)₃ to the mixture, followed by mixing; or other processes. In terms of easy operation, the acetalization reaction is preferably carried out by the process of adding a reaction mixture containing compound (III) after completion of the aldol reaction to HC(OR²)₃ and an acid catalyst, followed by mixing.

The acetalization reaction is carried out preferably in the range of from 0°C to 100°C, more preferably in the range of from 10°C to 40°C, particularly preferably in the range of from 20°C to 30°C, depending on the types of HC(OR³)₃ and the acid catalyst.

The reaction time is preferably from 10 minutes to 50 hours, more preferably from 30 minutes to 20 hours, particularly preferably from 1 to 10 hours, depending on the types of HC(OR³)₃ and the acid catalyst, and the reaction temperature.

The progress of the reaction can be determined by analytic means, such as thin layer chromatography, gas chromatography, and high performance liquid chromatography.

Compound (IV) contained in the reaction mixture thus obtained can be isolated by subjecting the reaction mixture to conventional post-treatments (e.g., neutralization, extraction, water washing, distillation, and crystallization) . Compound (IV) can be purified by subjecting compound (IV) to extraction purification treatment, distillation treatment, adsorption treatment with activated carbon, silica, alumina, and other materials, and chromatography treatment, such as silica gel column chromatography. Alternatively, compound (IV) may be subjected to the next step as being in the reaction mixture.

Step 3: Compound (IV) is reduced to give compound (V).

Compound (IV) is reduced by allowing compound (IV) to react with a reducing agent.

Examples of the reducing agent include sodium borohydride, sodium cyanoborohydride, lithium aluminum hydride, sodium aluminum hydride, diisobutyl aluminum hydride, and sodium bis(2-methoxyethoxy)aluminum hydride.

The amount of the reducing agent used is preferably from 1.0 to 3.0 mol, more preferably from 1.0 to 2.0 mol per mole of compound (IV) in terms of the yield and cost efficiency.

When sodium borohydride is used as a reducing agent, this reduction reaction is preferably carried out in the presence of acid or alcohol in terms of the reaction rate.

Examples of the acid include boron trifluoride-THF complex, boron trifluoride-diethyl ether complex, boron trichloride, sulfuric acid, and borane.

The amount of the acid used is preferably from 0.1 to 3.0 mol, more preferably from 0.5 to 2.0 mol per mole of compound (IV) in terms of the yield.

Examples of the alcohol include methanol, ethanol, 1-propanol, 2-propanol, and 2-methyl-2-propanol.

Regarding to the amount of alcohol, alcohol is preferably used as a solvent, or used in an amount of from 0.1 to 5.0 g, more preferably from 0.2 to 2.0 g per gram of compound (IV) in terms of the yield.

The reduction reaction is preferably carried out in the presence of a solvent. Examples of the solvent used in the pres ent invention include aromatic hydrocarbon solvents (e.g., toluene, benzene, xylene); aliphatic hydrocarbon solvents (e.g., hexane, heptane, cyclahexane) ; alcohol solvents (e.g., methanol, ethanol, 2-propanol, 2-methyl-2-propanol) ; ether solvents (e.g., diethyl ether, tetrahydrofuran, 2-methyltetrahydrofuran, 1,2-dimethoxyethane, tert-butyl methyl ether, tert-butyl ethyl ether, diisopropyl ether, dibutyl ether, cyclopentyl methyl ether, 1,4-dioxane, and bis(2-methoxyethyl)ether) ; water; and mixed solvents thereof.

Above all, aromatic hydrocarbon solvents, alcohol solvents, ether solvents, or mixed solvents containing at least one of these are preferred, although it depends on the reducing agent. Furthermore, methanol, ethanol, 2-propanol, 2-methyl-2-propanol, tetrahydrofuran, 2-methyltetrahydrofuran, toluene, or mixed solvents thereof are more preferred; and tetrahydrofuran or a mixed solvent of tetrahydrofuran and toluene is particularly preferred in terms of the reactivity.

The reduction reaction is carried out by a process of adding a reducing agent and a solvent to compound (IV) and mixing these; a process of mixing compound (IV) and a solvent and adding a reducing agent to the mixture, followed by mixing; a process of mixing a reducing agent and a solvent and adding compound (IV) to the mixture; or other processes. In terms of the yield and selectivity, preferred is the process of mixing compound (IV) and a solvent and adding a reducing agent to the mixture, followed by mixing. When sodium borohydride is used as a reducing agent, the reduction reaction is preferably carried out by a process of mixing compound (IV), sodium borohydride, and a solvent and adding an acid to the mixture, followed by mixing, or a process of mixing compound (IV), alcohol, and a solvent and adding sodium borohydride to the mixture, followed by mixing.

The reduction reaction is carried out preferably in the range of from -10°C to 80°C, more preferably in the range of from 0°C to 60°C, particularly preferably in the range of from 20°C to 50°C, depending on the types of the reducing agent and acid.

The reaction time is preferably from 10 minutes to 20 hours, more preferably from 30 minutes to 10 hours, particularly preferably from 1 to 6 hours, depending on the type of the reducing agent and the reaction temperature.

The progress of the reaction can be determined by analytic means, such as thin layer chromatography, gas chromatography, and high performance liquid chromatography.

Compound (V) contained in the reaction mixture thus obtained can be isolated by subjecting the reaction mixture to conventional post-treatments (e.g., neutralization, extraction, water washing, distillation, and crystallization) . Compound (V) can be purified by subjecting compound (V) to extraction purification treatment, distillation treatment, adsorption treatment with activated carbon, silica, alumina, and other materials, and chromatography treatment, such as silica gel column chromatography. Alternatively, compound (V) may be subjected to the next step as being in the reaction mixture.

Step 4: The acetalized formyl group represented by CH(OR³)₂ in compound (V) is deprotected so that the obtained product is cyclized to give compound (VI).

The cyclization reaction proceeds just after the acetalized formyl group represented by CH(OR³)₂ in compound (V) is deprotected.

The deprotection reaction is carried out by treating compound (V) with an acid.

Examples of the acid include inorganic proton acids, such as hydrochloric acid, sulfuric acid, and phosphoric acid; organic proton acids, such as methanesulfonic acid, pyridinium p-toluenesulfonate, p-toluenesulfonic acid or its hydrate (monohydrate), and acidic ion-exchange resin; and aprotic Lewis acids, such as boron trifluoride-THF complex, magnesium chloride, zinc chloride, and aluminum chloride. In terms of the yield, inorganic proton acids or organic proton acids are preferred; sulfuric acid or methanesulfonic acid is particularly preferred.

The amount of the acid used is preferably from 0.005 to 0.3 mol, more preferably from 0.02 to 0.1 mol per mole of compound (V) in terms of the yield and cost efficiency.

The reaction is preferably carried out in the presence of a solvent. Examples of the solvent used in the present invention include aromatic hydrocarbon solvents (e.g., toluene, benzene, xylene); aliphatic hydrocarbon solvents (e.g., hexane, heptane, cyclohexane); alcohol solvents (e.g., methanol, ethanol, 2-propanol, 2-methyl-2-propanol); halogenated hydrocarbon solvents (e.g., chloroform, dichloromethane, carbon tetrachloride, 1-chlorobutane); ether solvents (e.g., diethyl ether, tetrahydrofuran, 2-methyltetrahydrofuran, 1,2-dimethoxyethane, tert-butyl methyl ether, tert-butyl ethyl ether, diisopropyl ether, dibutyl ether, cyclopentyl methyl ether, 1,4-dioxane, and bis(2-methoxyethyl)ether); ester solvents (e.g., ethyl acetate, isopropyl acetate) ; ketone solvents (e.g., acetone, 2-butanone; 3-pentanone); nitrile solvents (e.g., acetonitrile, propionitrile); aprotic polar solvents (e.g., dimethylformamide, dimethylacetamide, dimethyl sulfoxide); and mixed solvents thereof.

Above all, alcohol solvents, ether solvents, or mixed solvents containing one or more of these are preferred, although it depends on the types of compound (V) and acid. Furthermore, methanol, ethanol, 2-propanol, tetrahydrofuran, 2-methyltetrahydrofuran, or mixed solvents thereof are more preferred; and methanol or tetrahydrofuran is particularly preferred in terms of the reactivity.

In this case, using a non-alcohol solvent provides compound (VI) where R⁴ = R³; using an alcohol solvent provides a mixture of compound (VI) where R⁴ = R³ and compound (VI) where R⁴ is the group corresponding to alcohol used,

The reaction is carried out by a process of mixing compound (V), an acid, and a solvent; a process of mixing compound (V) and a solvent and adding an acid to the mixture, followed by mixing; a process of mixing an acid and a solvent and adding compound (V) to the mixture, followed by mixing; or other processes. In terms of the yield and selectivity, the reaction is preferably carried out by the process of mixing compound (V) and a solvent and adding an acid to the mixture, followed by mixing.

The reaction is carried out preferably in the range of from -10°C to 50°C, more preferably in the range of from 0°C to 40°C, particularly preferably in the range of from 10°C to 30°C, depending on the types of the acid and solvent.

The reaction time is preferably from 5 minutes to 10 hours, more preferably from 10 minutes to 5 hours, particularly preferably from 30 minutes to 3 hours, depending on the types of the acid and solvent and the reaction temperature.

The progress of the reaction can be determined by analytic means, such as thin layer chromatography, gas chromatography, and high performance liquid chromatography.

Compound (VI) contained in the reaction mixture thus obtained can be isolated by subjecting the reaction mixture to conventional post-treatments (e.g., neutralization, extraction, water washing, distillation, and crystallization). Compound (VI) can be purified by subjecting compound (VI) to extraction purification treatment, distillation treatment, adsorption treatment with activated carbon, silica, alumina, and other materials, and chromatography treatment, such as silica gel column chromatography. Alternatively, compound (VI) may be subjected to the next step as being in the reaction mixture.

Step 5: The R²-protected hydroxyl group in compound (VI) is deprotected so that the obtained product is cyclized to give compound (VII).

The cyclization reaction proceeds by deprotecting the R²-protected hydroxyl group in compound (VI), followed by an optional acid treatment.

The deprotection can be carried out by an conventional method as described in T. W. Greene and P. G. M. Wuts, "Protective Groups in Organic Synthesis" (published byWiley-Interscience, 4th Edition, 2006) . Particularly when R² is a C₇₋₁₄ aralkyl group, the deprotection is carried out by allowing compound (VI) to undergo the reaction in the presence of a metal catalyst and a hydrogen source.

Examples of the metal catalyst include palladium on carbon, palladium black, palladium chloride, palladium hydroxide, rhodium on carbon, platinum oxide, platinum black, platinum-palladium, Raney nickel, and Raney cobalt.

The amount of the metal catalyst used is preferably from 0.001 to 0.5 g, more preferably from 0.05 to 0.15 g per gram of compound (VI) in terms of the yield and cost efficiency.

Examples of the hydrogen source include hydrogen gas, ammonium formate, and cyclohexadiene.

The amount of the hydrogen source used is preferably from 1 to 5 mol, more preferably from 1 to 3 mol per mole of compound (VI). In the case of hydrogen gas, it is usually used at a pressure of from 0.1 to 1.0 Mpa because of restriction on experimental equipment.

The reaction is preferably carried out in the presence of a solvent. Examples of the solvent used in the present invention include aromatic hydrocarbon solvents (e.g. , toluene, benzene, xylene); aliphatic hydrocarbon solvents (e.g., hexane, heptane, cyclohexane); alcohol solvents (e.g., methanol, ethanol); ether solvents (e.g., diethyl ether, tetrahydrofuran, 2-methyltetrahydrofuran, 1,2-dimethoxyethane, tert-butyl methyl ether, tert-butyl ethyl ether, diisopropyl ether, dibutyl ether, cyclopentyl methyl, ether, 1,4-dioxane, and bis(2-methoxyethyl)ether); ester solvents (e.g., ethyl acetate, isopropyl acetate); water; and mixed solvents thereof.

Above all, alcohol solvents, ether solvents, or mixed solvents containing one or more of these are preferred; furthermore, methanol, ethanol, 2-propanol, tetrahydrofuran, 2-methyltetrahydrofuran, cyclopentyl methyl ether, or mixed solvents thereof are more preferred, and tetrahydrofuran is particularly preferred in terms of the reactivity, although the preferred solvent depends on the type of compound (VI).

The reaction is carried out by a process of mixing compound (VI), a metal catalyst, and a solvent and adding a hydrogen source to the mixture, followed by mixing; a process of mixing a metal catalyst and a solvent and adding a hydrogen source to the mixture, followed by mixing, and then adding compound (VI) to the mixture; or other processes. In terms of the operability, the reaction is preferably carried out by the process of mixing compound (VI), a metal catalyst, and a solvent and adding a hydrogen source to the mixture, followed by mixing.

The reaction is carried out preferably in the range of from 0°C to 60°C, more preferably in the range of from 10°C to 50°C, particularly preferably in the range of from 20°C to 40°C, depending on the types of the metal catalyst, hydrogen source, and solvent.

The reaction time is preferably from 1 to 20 hours, more preferably from 1 to 10 hours, particularly preferably from 1 to 5 hours, depending on the types of the metal catalyst, hydrogen source, and solvent and the reaction temperature.

The progress of the reaction can be determined by analytic means, such as thin layer chromatography, gas chromatography, and high performance liquid chromatography.

Compound (VII) contained in the reaction mixture thus obtained can be isolated by subjecting the reaction mixture to conventional post-treatments (e.g., neutralization, extraction, water washing, distillation, and crystallization). Compound (VII) can be purified by subjecting compound (VII) to extraction purification treatment, distillation treatment, adsorption treatment with activated carbon, silica, alumina, and other materials, and chromatography treatment, such as silica gel column chromatography. Alternatively, compound (VII) may be subjected to the next step as being in the reaction mixture.

Step 6: The hydroxyl group of compound (VII) is protected to give compound (VIII).

The protection reaction is carried out by introducing a protective group into the hydroxyl group of compound (VII).

The protective group for the hydroxyl group is introduced by allowing compound (VII) to react with a protective group-introducing agent corresponding to PG in the presence of a base.

Examples of the base include inorganic bases, such as sodium carbonate, potassium carbonate, cesium carbonate, sodium hydrogen carbonate, and potassium hydrogen carbonate; organic bases, such as triethylamine, trimethylamine, diisopropylethylamine, pyridine, and 2,6-lutidine.

The amount of the base used is preferably from 1.0 to 6.0 mol, more preferably from 1.0 to 3.0 mol per mole of compound (VII).

Examples of the protective group-introducing agent include di(N-succinimidyl) carbonate, bis(4-nitrophenyl) carbonate, 4-nitrophenyl chloroformate, benzoyl chloride, 4-nitrobenzoyl chloride, and phthalic anhydride.

The amount of the protective group-introducing agent used is preferably from 1.0 to 5.0 mol, more preferably from 1.0 to 2.5 mol per mole of compound (VII).

The protection reaction is preferably carried but in the presence of a solvent. Examples of the solvent used in the present invention include aromatic hydrocarbon solvents (e.g., toluene, benzene, xylene); aliphatic hydrocarbon solvents (e.g., hexane, heptane, cyclohexane); halogenated hydrocarbon solvents (e.g., chloroform, dichloromethane, carbon tetrachloride, 1-chlorobutane) ; ether solvents (e.g., diethyl ether, tetrahydrofuran, 2-methyltetrahydrofuran, 1,2-dimethoxyethane, tert-butyl methyl ether, tert-butyl ethyl ether, diisopropyl ether, dibutyl ether, cyclopentyl methyl ether, 1,4-dioxane, and bis (2-methoxyethyl)ether) ; ester solvents (e.g., ethyl acetate, isopropyl acetate); ketone solvents (e.g., acetone, 2-butanone, 3-pentanone); nitrile solvents (e.g., acetonitrile, propionitrile); aprotic polar solvents (e.g., dimethylformamide, dimethylacetamide, dimethyl sulfoxide); and mixed solvents thereof.

Above all, ether solvents, or mixed solvents containing at least one of these are preferred although it depends on the protective group-introducing agent and the base, nitrile solvents. Furthermore, acetonitrile and tetrahydrofuran are more preferred; and acetonitrile is particularly preferred in terms of the reactivity.

The protection reaction is carried out by a process of mixing compound (VII), a protective group-introducing agent, and a solvent and adding a base to the mixture, followed by mixing; a process of mixing compound (VII), a base, and a solvent and adding a protective group-introducing agent to the mixture, followed by mixing; a process of mixing compound (VII) and a solvent and adding a base and a protective group-introducing agent together to the mixture; or other processes. In terms of the yield and selectivity, the protection reaction is preferably carried out by the process of mixing compound (VII), a protective group-introducing agent, and a solvent and adding a base to the mixture, followed by mixing.

The protection reaction is carried out preferably in the range of from 0°C to 80°C, more preferably in the range of from 10°C to 60°C, particularly preferably in the range of from 20°C to 50°C, depending on the types of the protective group-introducing agent, solvent, and base.

The reaction time is preferably from 1 to 50 hours, more preferably from 1 to 20 hours, particularly preferably from 1 to 10 hours, depending on the types of the protective group-introducing agent, solvent, and base, and the reaction temperature.

The progress of the reaction can be determined by analytic means, such as thin layer chromatography, gas chromatography, and high performance liquid chromatography.

Compound (VIII) contained in the reaction mixture thus obtained can be isolated by subjecting the reaction mixture to conventional post-treatments (e.g., neutralization, extraction, water washing, distillation, and crystallization). Compound (VIII) can be purified by subjecting compound (VIII) to recrystallization treatment, extraction purification treatment, distillation treatment, adsorption treatment with activated carbon, silica, alumina, and other materials, and chromatography treatment, such as silica gel column chromatography. EXAMPLES

The present invention will be described below in more detail by way of Examples.

### Example 1 Synthesis of ethyl

### (2R,3S)-2-hydroxy-3-dimethoxymethyl-5-benzyloxypentanoate

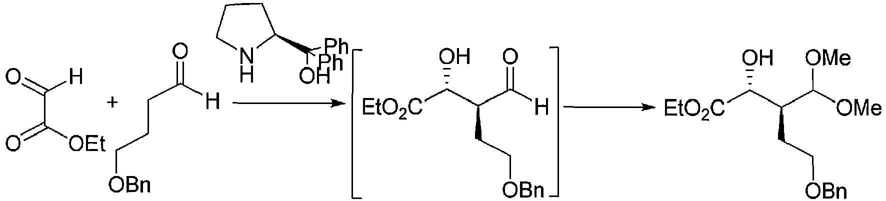

A solution (146.2 g) of ethyl glyoxylate (polymer) in toluene (0.67 mol, content 47%, toluene solution), 30.3 g (1.68 mol) of water, and 4.26 g (16.8 mmol) of (2S)-α,α-diphenyl-2-pyrrolidinemethanol were added to 125 g of THF, and 104.4 g (0.56 mol, content 95.8%) of 4-benzyloxybutanal was added dropwise to the mixture at room temperature and stirred at this temperature for 17 hours. Subsequently, 200 g of a 5% aqueous solution of NaCl was added for phase separation. The obtained organic layer was added dropwise to a mixture of 208.4 g (1.96 mol) of trimethyl orthoformate and 21.3 g (0.11 mol) of p-toluenesulfonic acid monohydrate at room temperature, and stirred at this temperature for two hours. This reaction mixture was added dropwise to 200 g of a 5% aqueous solution of NaHCO₃ at room temperature, and 350 g of toluene was added thereto for extraction. The aqueous layer was extracted again with 170 g of toluene, the organic layers were combined and the combined organic layer was washed with 100 g of a 5% aqueous solution of NaCl. The organic layer was dried over 100 g of anhydrous sodium sulfate and the solvent was then distilled off under reduced pressure to give 216.0 g of a liquid crude title compound. The content of this crude product was determined by HPLC (internal standard method) to obtain 73.5% yield (yield to 4-benzyloxy butanal) including diastereomers. The diastereomer ratio ((2R, 3S) form/ (2S,3S) form) in this case was about 15/1.

(Internal standard method: HPLC analysis conditions; column: MGIII (150 × 4.6 mm, 5 µm), Mobile phase A: 0.05% aqueous solution of trifluoroacetic acid (hereinafter referred to as TFA), Mobile phase B: 0.05% solution of TFA in acetonitrile, Flow rate: 1.0 ml/min, Detector: UV 215 nm).
¹H-NMR (CDCl₃, 400 MHz, major diastereomer) δppm: 7.34-7.27(5H, m), 4.56(1H, d, J=12.4 Hz), 4.51(1H, d, J=12.4 Hz), 4.36(1H, d, J=7.6 Hz), 4.26-4.17 (3H, m), 3.62-3.58 (2H, m), 3.32(3H, s), 3.31(3H, s), 2.51-2.45(1H, m), 1.96-1.72(2H, m), 1.31(3H, t, J=7.0 Hz)

### Example 2 Synthesis of ethyl

### (2R,3S)-2-hydroxy-3-dimethoxymethyl-5-benzyloxypentanoate

A solution (1.46 g) of ethyl glyoxylate (polymer) in toluene (6.73mmol, content 47%, toluene solution), 0.30 g (16.8 mmol) of water, and 42.6 mg (0.168 mmol) of (2S)-α,α-diphenyl-2-pyrrolidinemethanol were added to 1.25 g of THF, and 1.39 g (5.61 mmol, content 72%) of 4-benzyloxybutanal was added dropwise to the mixture at room temperature and stirred at this temperature for 23 hours. Subsequently, 2 g. of a 20% aqueous solution of NaCl was added for phase separation. The obtained organic layer was added dropwise to a mixture of 2.5 g of toluene, 2.38 g (22.44 mmol) of trimethyl orthoformate, and 53.4 mg (0.281 mmol) of p-toluenesulfonic acid monohydrate at room temperature, and stirred at this temperature for two hours. This reaction mixture was added dropwise to 2 g of a 5% aqueous solution of NaHCO₃ at room temperature, and 1 g of toluene was added thereto to separate and collect the organic layer. The aqueous layer was further extracted again with 2 g of toluene, and the organic layers were combined. The content of the title compound in this organic layer was determined by the method described in Example 1 to obtain 75.5% yield including diastereomers. The diastereomer ratio ((2R, 3S) form/ (2S, 3S) form) in this case was about 12/1. The enantiomer excess was determined by HPLC to obtain 93.8%ee.

HPLC conditions (enantiomer excess): Column: CHIRALPAK AS-RH(150 × 4.6 mm, 5 µm) + L-Column ODS (150 × 4.6 mm, 5 µm), Mobile phase A: acetonitrile, Mobile phase B: water, Flow rate; 1.0 ml/min, Detector: UV 220 nm.

### Examples 3 and 4 Synthesis of ethyl

### 2-hydroxy-3-d-imethoxymethyl-5-benzyloxy pentanoate

The same procedure as in Example 2 was carried out except that (2S)-*α*,*α*-diphenyl-2-pyrrolidinemethanol was changed to the catalysts indicated in Table 1 and the reaction time was extended. Table 2 shows the obtained results.

**[Table 1]**

| | EXAMPLE 3 | EXAMPLE 4 |
|---|---|---|
| CATALYST | 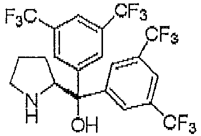 | 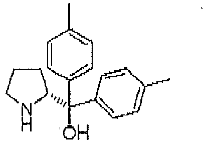 |
| REACTION TIME | 45 HOURS | 43 HOURS |

**[Table 2]**

| | EXAMPLE 3 | EXAMPLE 4 |
|---|---|---|
| PRODUCT | 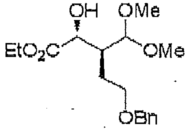 | 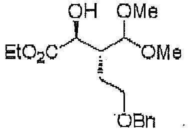 |
| YIELD | 71.1% | 72.0% |
| DIASTEREOMER RATIO | 7/1 | 9/1 |
| ENANTIOMER EXCESS | 96.5%ee | 91.4%ee |

### Example 5 Synthesis of

### (2R,3S)-3-dimethoxymathyl-5-benzyloxy-1,2-pentanediol

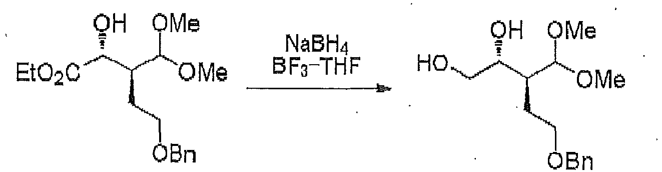

The crude ethyl
(2R,3S)-2-hydroxy-3-dimethoxymethyl-5-benzyloxypentanoate (162.2 g, 0.245 mol, content 49.3%) (a mixture with a diastereomer ratio of about 15/1) obtained in Example 1 was added to 344 g of THF, and 14.8 g (0.39 mol) of sodium borohydride was added thereto at room temperature. To the obtained mixture, 68.6 g (0.49 mol) of boron trifluoride tetrahydrofuran complex was added dropwise at this temperature, followed by washing with 12 g of THF. Subsequently, the reaction mass was stirred at room temperature for five hours. This reaction mixture was added dropwise to 512 g of a 10% aqueous solution of NH₄Cl at room temperature, and then washed well with 20 g of this aqueous solution. To the obtained mixture, 144 g of ethyl acetate was added for extraction. The aqueous layer was extracted again with 144 g of ethyl acetate, and the organic layers were combined and washed with 176 g of a mixture of a 2.5% aqueous solution of NaHCO₃ and a 10% aqueous solution of NaCl. The organic layer was concentrated, and 144 g of ethyl acetate was added to the organic layer, which was then washed again with 80 g of a 20% aqueous solution of NaCl. After the organic layer was dried over 40 g of anhydrous sodium sulfate, the solvent was distilled off to give 135.3 g of a liquid crude title compound (including the diastereomer) . When the content of this crude product was determined by HPLC (internal standard method) to obtain the yield, the title compound was quantitatively obtained in consideration of the diastereomer.
(For quantification: HPLC analysis conditions; Column: MGIII (150 × 4.6 mm, 5 µm), Mobile phase A: 10 mM aqueous solution of K₂HPO₄ (The pH was adjusted to 7 with H₃PO₄), Mobile phase B: acetonitrile solution, Flow rate: 1.0 ml/min, Detector: UV 215 nm).
¹H-NMR (CDCl₃, 400 MHz, major diastereomer) δppm: 7.37-7.27 (5H, m), 4.51(2H, s), 4.42(1H, d, J=4.4 Hz), 3.79-3.74(1H, m), 3.66(2H, t, J=5.0 Hz), 3.61-3.52(3H, m), 3.41(3H, s), 3.39(3H, s), 2.53(1H, t, J=6.6 Hz), 2.09-2.03(1H, m), 1.83-1.68(2H, m) Example 6 Synthesis of

### (2R, 3S)-3-dimethoxymethyl-5-benzyloxy-1,2-pentanediol

To 2.95 g of the crude ethyl
(2R,3S)-2-hydroxy-3-dimethoxymethyl-5-benzyloxypentanoate (a mixture with a diastereomer ratio of about 15/1)(ethyl (2R,3S)-2-hydroxy-3-dimethoxymethyl-5-benzyloxypentanoate, content 59.2%, 0.0054 mol) obtained in the same manner as in Example 1, 0.42 g of methanol and 3.32 g of toluene were added. To the obtained mixture, 0.35 g (0.0092 mol) of sodium borohydride was added at 40°C and then stirred at 40°C for eight hours. The content of the title compound (including diastereomers) in the reaction mixture was then determined by the method described in Example 5 to obtain 93.6% yield (including diastereomers).

### Example 7 Synthesis of

### (2R,3S)-3-dimethoxymethyl-5-benzyloxy-1,2-pentanediol

To 2.95 g of the crude ethyl
(2R,3S)-2-hydroxy-3-dimethoxymethyl-5-benzyloxypentanoate (a mixture with a diastereomer ratio of about 15/1)(ethyl (2R,3S)-2-hydroxy-3-dimethoxymethyl-5-benzyloxypentanoate, content 59.2%, 0.0054 mol) obtained in the same manner as in Example 1, 0.42 g of methanol, 1.26 g of THF, and 3.32 g of toluene were added. To the obtained mixture, 0.35 g (0.0092 mol) of sodium borohydride was added at 40°C and then stirred at 40°C for eight hours. The content of the title compound (including diastereomers) in the reaction mixture was then determined by the method described in Example 5 to obtain 93.3% yield (including diastereomers).

### Example 8 Synthesis of

### (2R,3S)-3-dimethoxymethyl-5-benzyloxy-1,2-pentanediol

To 2.60 g of the crude ethyl
(2R,3S)-2-hydroxy-3-dimethoxymethyl-5-benzyloxypentanoate (a mixture with a diastereomer ratio of about 15/1)(ethyl (2R,3S)-2-hydroxy-3-dimethoxymethyl-5-benzyloxypentanoate, content 67.1%, 0.0053 mol) obtained in the same manner as in Example 1, 1.88 g of ethanol and 2.24 g. of toluene were added. To the obtained mixture, 0.35 g (0.0092 mol) of sodium borohydride was added at 35°C and then stirred at 35°C for eight hours. The content of the title compound (including diastereomers) in the reaction mixture was then determined by the method described in Example 5 to obtain 90.7% yield (including diastereomers).

### Example 9 Synthesis of

### (2R,3S)-3-dimethoxymethyl-5-benzyloxy-1,2-pentanediol

To 2.60 g of the crude ethyl
(2R,3S)-2-hydroxy-3-dimethoxymethyl-5-benzyloxypentanoate (a mixture with a diastereomer ratio of about 15/1)(ethyl (2R,3S)-2-hydroxy-3-dimethoxymethyl-5-benzyloxypentanoate, content 67.1%, 0.0053 mol) obtained in the same manner as in Example 1, 2.50 g of ethanol and 1.62 g of toluene were added. To the obtained mixture, 0.35 g (0.0092 mol) of sodium borohydride was added at 35°C and then stirred at 35°C for eight hours. The content of the title compound (including diastereomers) in the reaction mixture was then determined by the method described in Example 5 to obtain 92.1% yield (including diastereomers).

### Example 10 Synthesis of

### (3R,4S)-tetrahydro-4-(2-benzyloxy)ethyl-5-methoxy-3-furanol

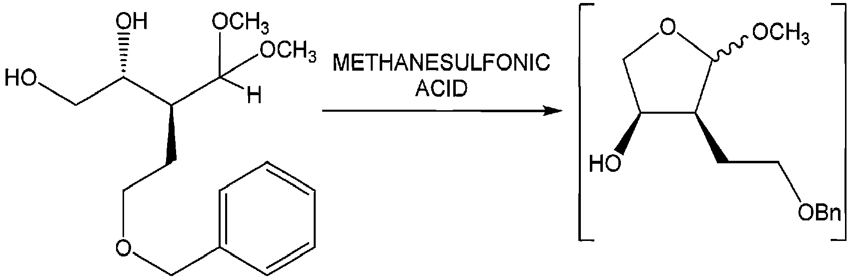

The crude
(2R,3S)-3-dimethoxymethyl-5-benzyloxy-1,2-pentanediol (5.0 g, pure content 2.35 g, 8.26 mmol) obtained in Example 5 was dissolved in methanol (6.76 g). To this solution, methanesulfonic acid (89.5 mg, 0.83 mmol) was added and stirred at 25°C for one hour, and sodium hydrogen carbonate (0.3 g) was then added to the mixture, which was then concentrated. To the obtained residue, ethyl acetate (50 mL) was added, and this solution was washed twice with a 5% aqueous solution (10 mL) of sodium chloride. The obtained organic layer was dried over anhydrous sodium sulfate, and the solvent was distilled off to give 2.0 g of a crude title compound (including diastereomers) .
¹H-NMR(CDCl₃, 400 MHz)δppm: 7.38-7.28(5H, m), 4.84(1/2H, d, J=4.4 Hz), 4.76(1/2H, d, J=4.4 Hz), 4.54(1H, s), 4.53(1H, s), 4.35-4.32(1/2H, m), 4.18-4.02(3/2H, m), 3.95(1/2H, d, J=10.0 Hz), 3.92(1/2H, d, J=10.0 Hz), 3.72-3.68.(1/2H, m), 3.63-3.48(3/2H, m), 3.39(3/2H, s), 3.34(3/2H, s), 3.13-3.12(1/2H, m), 2.85(1/2H, d, J=10.0 Hz), 2.18-1.94(2H, m), 1.92-1.82(1H, m).

### Example 11 Synthesis of

### (3R, 3aS, 6aR)-hexahydrofuro[2, 3-b]furan-3-ol

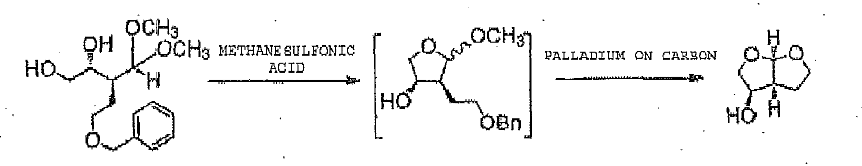

The crude
(2R,3S)-3-dimethoxymethyl-5-benzyloxy-1,2-pentanediol (127.3 g, content 68.7%, pure content 87.4 g, 0.307 mol) obtained in Example 5 was dissolved in tetrahydrofuran (100.1 g). To this solution, methanesulfonic acid (3.1 g, 0.031 mol) was added dropwise. This solution was stirred at 25°C for one hour to give crude
(3R,4S)-tetrahydro-4-(2-benzyloxy)ethyl-5-methoxy-3-furanol. Without isolation or purification, 10% palladium on carbon (containing 45% by weight of water, 20.5 g) was added to this reaction solution to undergo the reaction at from 25°C to 43°C at a hydrogen pressure of 0.5 MPa for three hours. After completion of the reaction, triethylamine (6.55 g, 0.65 mol) was added to this reaction solution, and insoluble matters were filtered out. After the filtrate was concentrated, toluene (100 ml) was added to the filtrate and the solvent was further distilled off to give a crude title compound (79.7 g). The content of this crude product was determined by GC (gas chromatography) (external standard method) to obtain 91.7% yield excluding the diastereomer. The diastereomer ratio ((3R, 3aS, 6aR) form/ (3S, 3aS, 6aR) form) in this case was 14/1, and the enantiomer excess determined by GC was 95%ee.

The GC measurement was conducted according to the following conditions.

GC conditions (quantification): DB-WAX 30 m × 0.53 mm 1 µm, 230°C
Temperature 100°C (3 min) → 5°C/min → 220°C (13 min), FID. GC conditions (enantiomer excess) : Astec G-TA 30 m × 0.25 mm, 0.125 µm, Column temperature 140°C, Analysis time 25 minutes, FID

### Example 12 Synthesis of

### (3R, 3aS, 6aR)-hexahydrofuro[2,3-b]furan-3-ol

The crude
(2R,3S)-3-dimethoxymethyl-5-benzyloxy-1,2-pentanediol (111.8 g, content 72.1%, pure content 80.6 g, 0.283 mol) obtained in the same manner as in Example 5 was dissolved in tetrahydrofuran (206.2 g). To this solution, sulfuric acid (0.9 g, 0.009 mol) was added dropwise.. This solution was stirred at 25°C for three hours to give crude (3R, 4S)-tetrahydro-4-(2-benzyloxy)ethyl-5-methoxy-3-furanol. Without isolating or purifying this product, 10% palladium on carbon (containing 54% by weight of water, 4.1 g) and sulfuric acid (0.6 g, 0.006 mol) were added to this reaction solution to undergo the reaction at from 25°C to 37°C at a hydrogen pressure of 0.5 MPa for three hours. After completion of the reaction, a solution of sodium methoxide in methanol (11.18 g, 0.058 mol) and methanol (16.5 g) were added to this reaction solution, and celite was used for precoating to filter insoluble matters. The insoluble matters were washed with tetrahydrofuran (82.5 g), and the filtrate combined with the washing liquid was concentrated to give a crude title compound (64.6 g). The content of this crude product was determined by GC (gas chromatography) (internal standard method) to obtain 74% yield excluding the diastereomer. The diastereomer ratio ((3R, 3aS, 6aR) form/(3S, 3aS, 6aR) form) in this case was 12/1.

The GC measurement here was conducted according to the following conditions.

GC conditions (quantification): DB-5 30 m × 0.25 mm 0.25 µm, Inj. temperature 260°C, Column temperature 60°C (5 min) → 5°C/min → 250°C (10 min) Det. temperature 260°C (FID)

### Example 13

### 1-[[[[(3R,3aS,6aR)-hexahydrofuro[2,3-b]furan-3-yl] oxy]carbonyl]oxy]pyrrolidine-2,5-dione

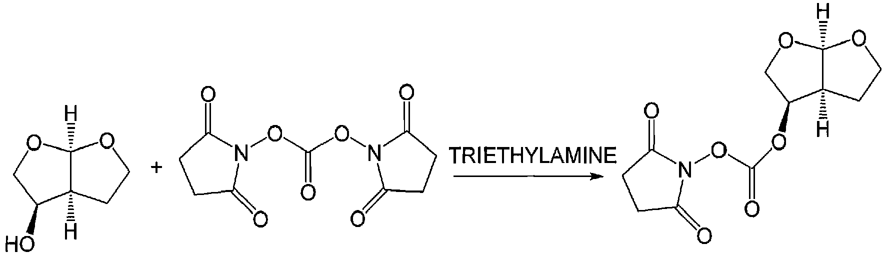

Di(N-succinimidyl) carbonate (14.5 g, 0.057 mol) and the oily product (5.0 g, 0.038 mol) obtained in Example 11 were suspended in acetonitrile (61.4 g). To this suspension, triethylamine (6.49 g, 0.064 mol) was added dropwise at 24°C. The reaction mixture was heated to 45°C and stirred for one hour, and then the solvent was distilled off. To the residue, ethyl acetate (125 mL) and a 20% salt solution (20 mL) were added. After insoluble solids were filtered out, the filtrate was subjected to liquid-liquid extraction. Furthermore, a 20% salt solution (20 mL) was added to the organic layer for phase separation. The organic layer was dried over anhydrous sodium sulfate (15.0 g) and the solvent was distilled off. To the residue thus obtained, tetrahydrofuran and heptane were added to undergo crystallization, providing 6.0 g of the title compound in a pale brownish white crystal (56% yield). The diastereomer ratio ((3R,3aS,6aR) form/(3S,3aS,6aR) form) determined by NMR analysis was 97/3, and the result of the enantiomer excess determined by HPLC was 99.5%ee.

HPLC conditions (enantiomer excess): Column: CHIRALPAK IA (250 × 4.6 mm, 5 µm), Mobile phase A: hexane, Mobile phase B: tetrahydrofuran, Flow rate: 0.5 ml/min, Detector: UV 225 nm. ¹H-NMR(CDCl₃,400 MHz)δppm: 1.93-2.04(m, 1H), 2.12-2.18(m, 1H), 2.84(s, 4H), 3.08-3.16(m, 1H), 3.91-3.97(m, 2H), 4.04(dt, 1H, J=2.4, 8.4 Hz), 4.12(dd, 1H, J=6.0, 9.6 Hz), 5.25(td, 1H, J=8.4, 5,6 Hz), 5.75(d, 1H, J=4.8 Hz)

### INDUSTRIAL APPLICABILITY

The present invention can produce compound (VII) having a good diastereomer ratio and enantiomer excess efficiently and inexpensively on an industrial scale. Compound (VIII) having a better diastereomer ratio and enantiomer excess can be derived from Compound (VII).

## Claims

1. A method for producing a compound represented by formula (V) 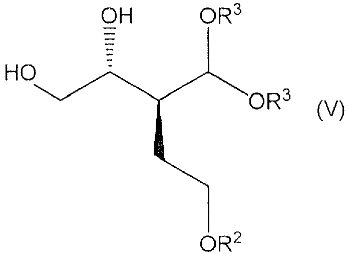 wherein R² represents a protective group of a hydroxyl group, and R³ represents a C₁₋₁₂ alkyl group, C₂₋₁₂ alkenyl group, C₂₋₁₂ alkynyl group, or C₇₋₁₄ aralkyl group,
the method comprising the steps of:
allowing a compound represented by formula (I), 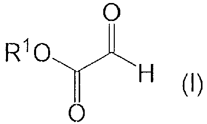 wherein R¹ represents a protective group of a carboxyl group, or a polymer thereof to react with a compound represented by formula (II), 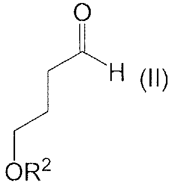 wherein R² has the same meaning as defined above,
in the presence of an optically active amine catalyst to give a compound represented by formula (III), 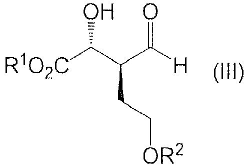 wherein R¹ and R² have the same meaning as defined above;
acetalizing the compound represented by formula (III) to give a compound represented by formula (IV), 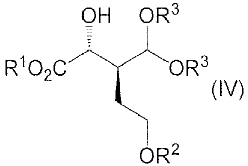 wherein R¹, R², and R³ have the same meaning as defined above; and
reducing the compound represented by formula (IV) to give the compound represented by formula (V).

2. A method for producing a compound represented by formula (VII), 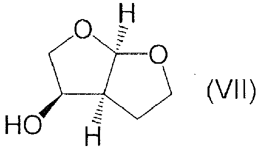 the method comprising the steps of:
producing a compound represented by formula (V) according to a method as defined in claim 1; and
deprotecting the acetalized formyl group represented by CH(OR³)₂ and the R²-protected hydroxyl group in the compound represented by formula (V) so that the obtained product is cyclized to give the compound represented by formula (VII).

3. A method according to claim 2 comprising the steps of:
producing a compound represented by formula (V) according to a method as defined in claim 1; and
deprotecting the acetalized formyl group represented by CH(OR³)₂ in the compound represented by formula (V) so that the obtained product is cyclized to give a compound represented by formula (VI), 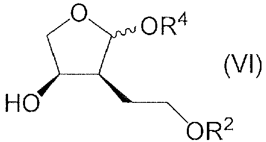 wherein R⁴ represents a C₁₋₁₂ alkyl group, C₂₋₁₂ alkenyl group, C₂₋₁₂ alkynyl group, or C₇₋₁₄ aralkyl group, and R² has the same meaning as defined above; and
deprotecting the R²-protected hydroxyl group in the compound represented by formula (VI) so that the obtained product is cyclized to give the compound represented by formula (VII).

4. A method for producing a compound represented by formula (VI), 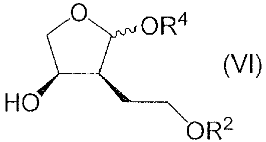 wherein R⁴ represents a C₁₋₁₂ alkyl group, C₂₋₁₂ alkenyl group, C₂₋₁₂ alkynyl group, or C₇₋₁₄ aralkyl group, and R² represents a protective group of a hydroxyl group,
the method comprising the steps of:
producing a compound represented by formula (V) according to a method as defined in claim 1; and
deprotecting the acetalized formyl group represented by CH(OR³)₂ in the compound represented by formula (V) so that the obtained product is cyclized to give the compound represented by formula (VI).

5. A method for producing a compound represented by formula (VIII), 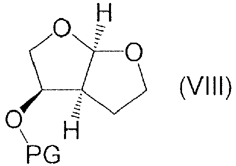 wherein PG represents a protective group of a hydroxyl group, the method comprising the steps of:
producing a compound represented by formula (V) according to a method as defined in claim 1; and
deprotecting the acetalized formyl group represented by CH(OR³)₂ in the compound represented by formula (V) so that the obtained product is cyclized to give a compound represented by formula (VI), 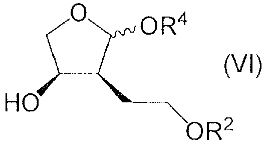 wherein R⁴ represents a C₁₋₁₂ alkyl group, C₂₋₁₂ alkenyl group, C₂₋₁₂ alkynyl group, or C₇₋₁₄ aralkyl group, and R² has the same meaning as defined above;
deprotecting the R²-protected hydroxyl group in the compound represented by formula (VI) so that the obtained product is cyclized to give a compound represented by formula (VII); and 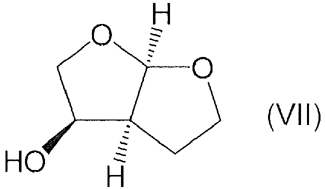
protecting the hydroxyl group of the compound represented by formula (VII) to give the compound represented by formula (VIII).

6. The production method according to any one of claims 1 to 5, wherein the optically active amine catalyst is a compound represented by formula (IX), 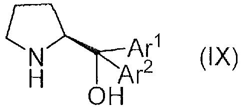 wherein Ar¹ and Ar² each independently represent a C₁₋₁₂ alkyl group, C₂₋₁₂ alkenyl group, C₂₋₁₂ alkynyl group, C₇₋₁₄ aralkyl group, or optionally substituted phenyl group.

7. A compound which is
a) represented by formula (III), 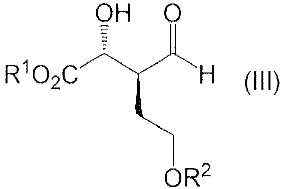 wherein R¹ represents a protective group of a carboxyl group, and R² represents a protective group of a hydroxyl group; or
b) represented by formula (IV), 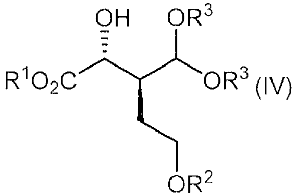 wherein R¹ represents a protective group of a carboxyl group, R² represents a protective group of a hydroxyl group, and R³ represents a C₁₋₁₂ alkyl group, C₂₋₁₂ alkenyl group, C₂₋₁₂ alkynyl group, or C₇₋₁₄ aralkyl group; or
c) represented by formula (V), 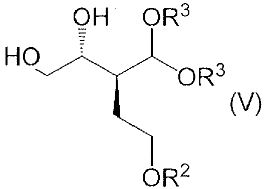 wherein R² represents a protective group of a hydroxyl group, and R³ represents a C₁₋₁₂ alkyl group, C₂₋₁₂ alkenyl group, C₂₋₁₂ alkynyl group, or C₇₋₁₄ aralkyl group; or
d) represented by formula (VI), 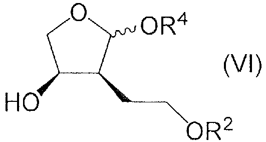 wherein R² represents a protective group of a hydroxyl group, and R⁴ represents a C₁₋₁₂ alkyl group, C₂₋₁₂ alkenyl group, C₂₋₁₂ alkynyl group, or C₇₋₁₄ aralkyl group.

8. The compound according to claim 7 which is represented by formula (III) and wherein R¹ is an ethyl group and R² is a benzyl group.

9. The compound according to claim 7 which is represented by formula (IV) and wherein R¹ is an ethyl group, R² is a benzyl group and R³ is a methyl group.

10. The compound according to claim 7 which is represented by formula (V) and wherein R² is a benzyl group and R³ is a methyl group.

11. The compound according to claim 7 which is represented by formula (VI) and wherein R² is a benzyl group and R⁴ is a methyl group.

## Patentansprüche

1. Ein Verfahren zur Herstellung einer Verbindung, dargestellt durch Formel (V): 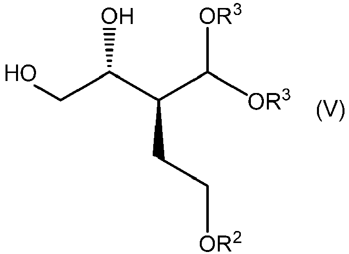 wobei R² eine Schutzgruppe einer Hydroxylgruppe darstellt und R³ eine C₁₋₁₂-Alkylgruppe, C₂₋₁₂-Alkenylgruppe, C₂₋₁₂-Alkinylgruppe oder C₇₋₁₄-Aralkylgruppe darstellt,
wobei das Verfahren die Schritte umfasst:
Reagieren lassen einer Verbindung dargestellt durch Formel (I), 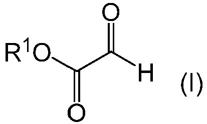
wobei R¹ eine Schutzgruppe einer Carboxylgruppe darstellt,
oder eines Polymers davon mit einer Verbindung dargestellt durch die Formel (II), 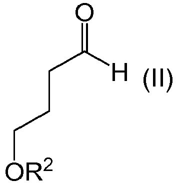
wobei R² die vorstehend definierte Bedeutung hat,
in Gegenwart eines optisch aktiven Aminkatalysators, um eine Verbindung dargestellt durch die Formel (III) zu ergeben, 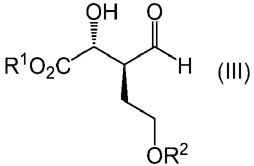
wobei R¹ und R² die gleiche Bedeutung wie vorstehend definiert haben;
Acetalisierung der Verbindung dargstellt durch Formel (III), um eine Verbindung dargestellt durch Formel (IV) zu ergeben, 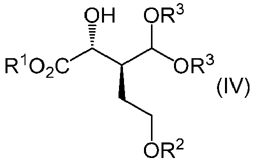
wobei R¹, R² und R³ die gleiche Bedeutung wie vorstehend definiert haben; und
Reduzieren der Verbindung dargestellt durch Formel (IV), um die Verbindung dargestellt durch Formel (V) zu ergeben.

2. Ein Verfahren zur Herstellung einer Verbindung, dargestellt durch Formel (VII): 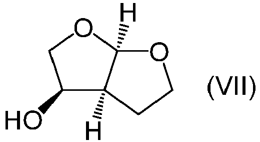 wobei das Verfahren die Schritte umfasst:
Herstellen einer Verbindung dargestellt durch Formel (V) gemäß einem Verfahren wie in Anspruch 1 definiert; und
Entschützen der acetalisierten Formylgruppe dargestellt durch CH(OR³)₂ und der R²-geschützten Hydroxylgruppe in der Verbindung dargestellt durch Formel (V), so dass das erhaltene Produkt cyclisiert wird, um die Verbindung dargestellt durch Formel (VII) zu ergeben.

3. Ein Verfahren gemäß Anspruch 2, umfassend die Schritte:
Herstellen einer Verbindung dargestellt durch Formel (V) gemäß einem Verfahren wie in Anspruch 1 definiert; und
Entschützen der acetalisierten Formylgruppe dargestellt durch CH(OR³)₂ in der Verbindung dargestellt durch Formel (V), so dass das erhaltene Produkt cyclisiert wird, um eine Verbindung dargestellt durch Formel (VI) zu ergeben. 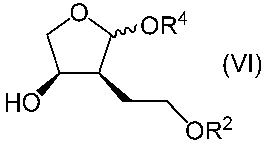
wobei R⁴ eine C₁₋₁₂-Alkylgruppe, C₂₋₁₂-Alkenylgruppe, C₂₋₁₂-Alkinylgruppe oder C₇₋₁₄-Aralkylgruppe darstellt und R² die gleiche Bedeutung wie oben definiert hat; und
Entschützen der R²-geschützten Hydroxylgruppe in der Verbindung dargestellt durch Formel (VI), so dass das erhaltene Produkt cyclisiert wird, um die Verbindung dargestellt durch Formel (VII) zu ergeben.

4. Ein Verfahren zur Herstellung einer Verbindung, dargestellt durch Formel (VI): 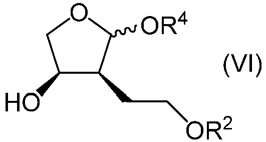
wobei R⁴ eine C₁₋₁₂-Alkylgruppe, C₂₋₁₂-Alkenylgruppe, C₂₋₁₂-Alkinylgruppe oder C₇₋₁₄-Aralkylgruppe darstellt und R² eine Schutzgruppe einer Hydroxylgruppe darstellt,
wobei das Verfahren die Schritte umfasst:
Herstellen einer Verbindung dargestellt durch Formel (V) gemäß einem Verfahren wie in Anspruch 1 definiert; und
Entschützen der acetalisierten Formylgruppe dargestellt durch CH(OR³)₂ in der Verbindung dargestellt durch Formel (V), so dass das erhaltene Produkt cyclisiert wird, um die Verbindung dargestellt durch Formel (VI) zu ergeben.

5. Ein Verfahren zur Herstellung einer Verbindung, dargestellt durch Formel (VIII): 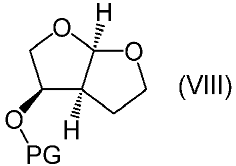 wobei PG eine Schutzgruppe einer Hydroxylgruppe darstellt,
wobei das Verfahren die Schritte umfasst:
Herstellen einer Verbindung dargestellt durch Formel (V) gemäß einem Verfahren wie in Anspruch 1 definiert; und
Entschützen der acetalisierten Formylgruppe dargestellt durch CH(OR³)₂ in der Verbindung dargestellt durch Formel (V), so dass das erhaltene Produkt cyclisiert wird, um eine Verbindung dargestellt durch Formel (VI) zu ergeben. 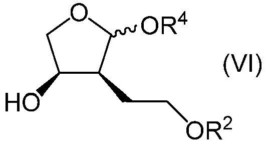
wobei R4 eine C1-12-Alkylgruppe, C2-12-Alkenylgruppe, C2-12-Alkinylgruppe oder C7-14-Aralkylgruppe darstellt und R2 die gleiche Bedeutung wie oben definiert hat;
Entschützen der R²-geschützten Hydroxylgruppe in der Verbindung dargestellt durch Formel (VI), so dass das erhaltene Produkt cyclisiert wird, um eine Verbindung dargestellt durch Formel (VII) zu ergeben; und 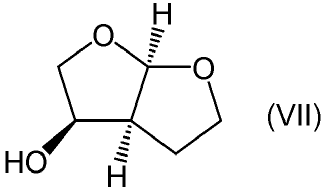
Schützen der Hydroxylgruppe der Verbindung dargestellt durch Formel (VII), um die Verbindung dargestellt durch Formel (VIII) zu ergeben.

6. Das Herstellungsverfahren gemäß einem der Ansprüche 1 bis 5, wobei der optisch aktive Aminkatalysator eine Verbindung dargestellt durch Formel (IX) ist, 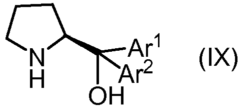 wobei Ar¹ und Ar² jeweils unabhängig eine C₁₋₁₂-Alkylgruppe, C₂₋₁₂-Alkenylgruppe, C₂₋₁₂-Alkinylgruppe, C₇₋₁₄-Aralkylgruppe oder gegebenenfalls substituierte Phenylgruppe darstellen.

7. Eine Verbindung, welche
a) durch Formel (III) dargestellt ist, 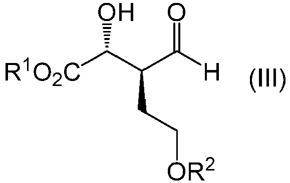 wobei R¹ eine Schutzgruppe einer Carboxylgruppe darstellt und R² eine Schutzgruppe einer Hydroxylgruppe darstellt; oder
b) durch Formel (IV) dargestellt ist, 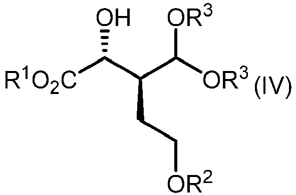 wobei R¹ eine Schutzgruppe einer Carboxylgruppe darstellt, R² eine Schutzgruppe einer Hydroxylgruppe darstellt und R³ eine C₁₋₁₂-Alkylgruppe, C₂₋₁₂-Alkenylgruppe, C₂₋₁₂-Alkinylgruppe oder C₇₋₁₄-Aralkylgruppe darstellt; oder
c) durch Formel (V) dargestellt ist, 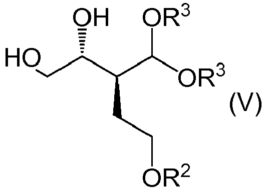 wobei R² eine Schutzgruppe einer Hydroxylgruppe darstellt und R³ eine C₁₋₁₂-Alkylgruppe, C₂₋₁₂-Alkenylgruppe, C₂₋₁₂-Alkinylgruppe oder C₇₋₁₄-Aralkylgruppe darstellt; oder
d) durch Formel (VI) dargestellt ist, 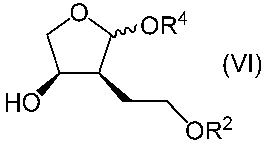 wobei R² eine Schutzgruppe einer Hydroxylgruppe darstellt und R⁴ eine C₁₋₁₂-Alkylgruppe, C₂₋₁₂-Alkenylgruppe, C₂₋₁₂-Alkinylgruppe oder C₇₋₁₄-Aralkylgruppe darstellt.

8. Die Verbindung gemäß Anspruch 7, welche durch Formel (III) dargestellt ist und wobei R¹ eine Ethylgruppe ist und R² eine Benzylgruppe ist.

9. Die Verbindung gemäß Anspruch 7, welche durch Formel (IV) dargestellt ist und wobei R¹ eine Ethylgruppe ist, R² eine Benzylgruppe ist und R³ eine Methylgruppe ist.

10. Die Verbindung gemäß Anspruch 7, welche durch Formel (V) dargestellt ist und wobei R² eine Benzylgruppe ist und R³ eine Methylgruppe ist.

11. Die Verbindung gemäß Anspruch 7, welche durch Formel (VI) dargestellt ist und wobei R² eine Benzylgruppe ist und R⁴ eine Methylgruppe ist.

## Revendications

1. Procédé de production d'un composé représenté par la formule (V) 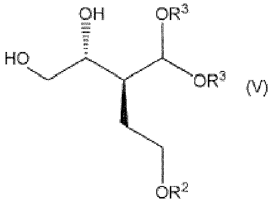 où R² représente un groupe protecteur d'un groupe hydroxyle, et R³ représente un groupe alkyle en C₁₋₁₂, un groupe alcényle en C₂₋₁₂, un groupe alcynyle en C₂₋₁₂, ou un groupe aralkyle en C₇₋₁₄,
le procédé comprenant les étapes de :
laisser un composé représenté par la formule (I), 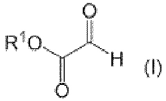 où R¹ représente un groupe protecteur d'un groupe carboxyle, ou un polymère de celui-ci réagir avec un composé représenté par la formule (II), 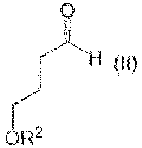
où R² a la même signification que définie ci-dessus,
en présence d'un catalyseur d'amine optiquement actif pour fournir un composé représenté par la formule (III), 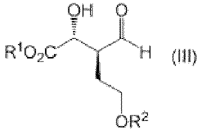
où R¹ et R² ont les mêmes significations que définies ci-dessus ;
acétalisation du composé représenté par la formule (III) pour fournir un composé représenté par la formule (IV), 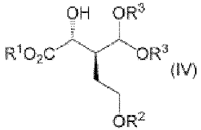
où R¹, R², et R³ ont les mêmes significations que définies ci-dessus ; et
réduction du composé représenté par la formule (IV) pour fournir le composé représenté par la formule (V).

2. Procédé de production d'un composé représenté par la formule (VII), 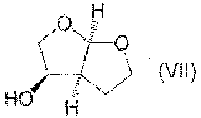 le procédé comprenant les étapes de :
production d'un composé représenté par la formule (V) selon un procédé comme défini dans la revendication 1 ; et
déprotection du groupe formyle acétalisé représenté par CH(OR³)₂ et du groupe hydroxyle R²-protégé dans le composé représenté par la formule (V) de sorte que le produit obtenu est cyclisé pour fournir le composé représenté par la formule (VII).

3. Procédé selon la revendication 2 comprenant les étapes de :
production d'un composé représenté par la formule (V) selon un procédé comme défini dans la revendication 1 ; et
déprotection du groupe formyle acétalisé représenté par CH(OR³)₂ dans le composé représenté par la formule (V) de sorte que le produit obtenu est cyclisé pour fournir un composé représenté par la formule (VI), 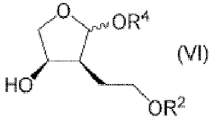
où R⁴ représente un groupe alkyle en C₁₋₁₂, un groupe alcényle en C₂₋₁₂, un groupe alcynyle en C₂₋₁₂, ou un groupe aralkyle en C₇₋₁₄, et R² a la même signification que définie ci-dessus ; et
la déprotection du groupe hydroxyle R²-protégé dans le composé représenté par la formule (VI) de sorte que le produit obtenu est cyclisé pour fournir le composé représenté par la formule (VII).

4. Procédé de production d'un composé représenté par la formule (VI), 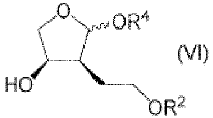 où R⁴ représente un groupe alkyle en C₁₋₁₂, un groupe alcényle en C₂₋₁₂, un groupe alcynyle en C₂₋₁₂, ou un groupe aralkyle en C₇₋₁₄, et R² représente un groupe protecteur d'un groupe hydroxyle,
le procédé comprenant les étapes de :
production d'un composé représenté par la formule (V) selon un procédé comme défini dans la revendication 1 ; et
déprotection du groupe formyle acétalisé représenté par CH(OR³)₂ dans le composé représenté par la formule (V) de sorte que le produit obtenu est cyclisé pour fournir le composé représenté par la formule (VI).

5. Procédé de production d'un composé représenté par la formule (VIII), 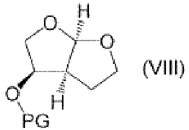 où PG représente un groupe protecteur d'un groupe hydroxyle, le procédé comprenant les étapes de :
production d'un composé représenté par la formule (V) selon un procédé comme défini dans la revendication 1 ; et
déprotection du groupe formyle acétalisé représenté par CH(OR³)₂ dans le composé représenté par la formule (V) de sorte que le produit obtenu est cyclisé pour fournir un composé représenté par la formule (VI), 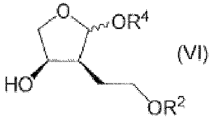
où R⁴ représente un groupe alkyle en C₁₋₁₂, un groupe alcényle en C₂₋₁₂, un groupe alcynyle en C₂₋₁₂, ou un groupe aralkyle en C₇₋₁₄, et R² a la même signification que définie ci-dessus ;
déprotection du groupe hydroxyle R²-protégé dans le composé représenté par la formule (VI) de sorte que le produit obtenu est cyclisé pour fournir un composé représenté par la formule (VII) ; et 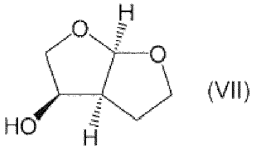
protection du groupe hydroxyle du composé représenté par la formule (VII) pour fournir le composé représenté par la formule (VIII).

6. Procédé de production selon l'une quelconque des revendications 1 à 5, dans lequel le catalyseur d'amine optiquement actif est un composé représenté par la formule (IX), 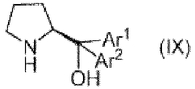 où Ar¹ et Ar² représentent chacun indépendamment un groupe alkyle en C₁₋₁₂, un groupe alcényle en C₂₋₁₂, un groupe alcynyle en C₂₋₁₂, un groupe aralkyle en C₇₋₁₄, ou un groupe phényle éventuellement substitué.

7. Composé qui est
a) représenté par la formule (III), 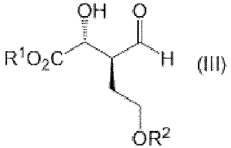 où R¹ représente un groupe protecteur d'un groupe carboxyle, et R² représente un groupe protecteur d'un groupe hydroxyle ; ou
b) représenté par la formule (IV), 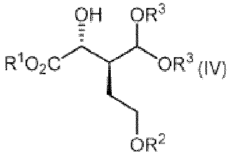 où R¹ représente un groupe protecteur d'un groupe carboxyle, R² représente un groupe protecteur d'un groupe hydroxyle, et R³ représente un groupe alkyle en C₁₋₁₂, un groupe alcényle en C₂₋₁₂, un groupe alcynyle en C₂₋₁₂, ou un groupe aralkyle en C₇₋₁₄ ; ou
c) représenté par la formule (V), 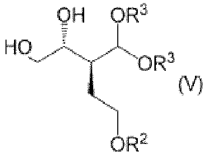 où R² représente un groupe protecteur d'un groupe hydroxyle, et R³ représente un groupe alkyle en C₁₋₁₂, un groupe alcényle en C₂₋₁₂, un groupe alcynyle en C₂₋₁₂, ou un groupe aralkyle en C₇₋₁₄; ou
d) représenté par la formule (VI), 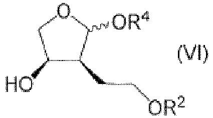 où R² représente un groupe protecteur d'un groupe hydroxyle, et R⁴ représente un groupe alkyle en C₁₋₁₂, un groupe alcényle en C₂₋₁₂, un groupe alcynyle en C₂₋₁₂, ou un groupe aralkyle en C₇₋₁₄.

8. Composé selon la revendication 7, lequel est représenté par la formule (III) et où R¹ est un groupe éthyle et R² est un groupe benzyle.

9. Composé selon la revendication 7, lequel est représenté par la formule (IV) et où R¹ est un groupe éthyle, R² est un groupe benzyle et R³ est un groupe méthyle.

10. Composé selon la revendication 7, lequel est représenté par la formule (V) et où R² est un groupe benzyle et R³ est un groupe méthyle.

11. Composé selon la revendication 7, lequel est représenté par la formule (VI) et où R² est un groupe benzyle et R⁴ est un groupe méthyle.
